# EUROPÄISCHE PATENTSCHRIFT

(11) **EP 0 711 835 B1**
(45) Veröffentlichungstag und Bekanntmachung des Hinweises auf die Patenterteilung: **07.07.2004**
(21) Anmeldenummer: 95890202.5
(22) Anmeldetag: 09.11.1995
(51) Int. Cl.: C12N 15/62, C12N 15/65, C12N 15/67, C12N 15/85, C12N 5/10, C12N 9/64, C12N 9/74, C07K 14/765, C07K 14/755, A61K 38/37, A61K 38/43

(54) **Selektion und Expression von Fremdproteinen mittels eines Selektions-Amplifikations-Systems**
Selection and expression of heterologous proteins using a selection-amplification-system
Sélection et expression des protéins hétérologues utilisant un système amplification-sélection

(30) Priorität: 14.11.1994 AT 209994
(43) Veröffentlichungstag der Anmeldung: 15.05.1996
(73) Patentinhaber: Baxter Aktiengesellschaft, 1221 Wien (AT)
(72) Erfinder: Herlitschka, Sabine E., Dr. Dipl.-Ing., A-5020 Salzburg (AT); Schlokat, Uwe, Dr., A-2304 Orth/Donau (AT); Falkner, Falko-Günther, Dr., A-2304 Orth/Donau (AT); Dorner, Friedrich, Prof. Dr., A-1230 Wien (AT)
(74) Vertreter: Pawloy, Peter Michael, Dipl.-Ing.

(56) Entgegenhaltungen:
- EP-A- 0 294 910
- WO-A-90/01550
- WO-A-92/08796
- WO-A-93/03143
- WO-A-94/05785
- WO-A-94/24870
- J. BIOL. CHEM., Bd. 263, Nr. 13, 5.Mai 1988 AM. SOC. BIOCHEM. MOL.BIOL.,INC.,BALTIMORE,US, Seiten 6352-6362, R.J. KAUFMAN ET AL. 'Synthesis, processing, and secretion of recombinant human factor VIII expressed in mammalian cells'
- BIOTECHNOLOGY, Bd. 12, Nr. 7, Juli 1994 NATURE PUBL. CO.,NEW YORK, US, Seiten 694-698, Y. SUGIMOTO ET AL. 'Efficient expression of drug-selectable genes in retroviral vectors under control of an internal ribosome entry site'
- FASEB JOURNAL, Bd. 4, Nr. 5, März 1990 FASEB,BATHESDA,MD,US, Seiten 1501-1507, U.A. GERMANN ET AL. 'Retroviral transfer of a chimeric multidrug resistance-adenosine deaminase gene'
- Herlitschka, S.: Dissertation, Universität für Bodenkultur, Wien, 1994
- Zusammenfassung der Dissertation: Herlitschka, Universität für Bodenkultur, Wien, 1994, Österreichische Dissertationsdatenbank
- EGHBALI B. ET AL: 'Expression of gap junction channels in communication-incompetent cells' PROC NATL ACAD SCI USA Bd. 87, Nr. 4, Februar 1990, Seiten 1328 - 1331

## Beschreibung

Die Erfindung betrifft Expressionsplasmide, die eine dicistronische Transkriptions-/Translationseinheit enthalten.

Die Expression von Proteinen in eukaryontischen Zellsystemen ist ein gängiges Verfahren in der Biotechnologie geworden. Die meistverwendeten Plasmidvektoren wurden für die effiziente Expression von Fremdproteinen konstruiert und enthalten unter anderem folgende genetische Elemente: einen bakteriellen Replikationsursprung ("Origin of Replication", ori), einen eukaryontischen Promotor für die Transkriptionsinitiation des Fremdgens, eukaryontische mRNA-Prozessierungssignale, Polylinker, welche multiple Restriktions-Endonuclease-Schnittstellen für die Insertion der Fremd-DNA beinhalten, und Selektions- und Amplifikationsmarker für die Selektion und Identifikation von Zellen, die die transfizierte DNA aufgenommen haben.

Der Selektionsmarker vermittelt der Zielzelle die Fähigkeit, in einem gegebenen Medium zu überleben. Dies kann durch Supplementieren einer fehlenden Stoffwechselfunktion geschehen oder durch die Eigenschaft, trotz Präsenz eines toxischen Agens zu wachsen.

Rezessive Resistenzgene können nur in solchen Wirtssystemen verwendet werden, welche hinsichtlich der untersuchten Selektionsaktivität defizient sind. Das Dihydrofolat-Reduktase-Gen (dhfr) ist der am meisten verwendete rezessive Selektionsmarker. Seine effiziente Verwendung ist auf dhfr-defiziente CHO-Zellen beschränkt. Die Dihydrofolat-Reduktase katalysiert die Reduktion von Folat zu Tetrahydrofolat (FH₄). FH₄ seinerseits wird für die Biosynthese von Glycin aus Serin, Thymidin-Monophosphat aus Deoxyuridin-Monophosphat und für die Purin-Biosynthese benötigt. Methotrexat (MTX), ein Folat-Analogon, bindet und inhibiert die Dihydrofolat-Reduktase und bewirkt damit den Zelltod der exponierten Zellen.

Dominante Resistenzgene finden unabhängig vom Genotyp des Wirtssystems Anwendung und sind somit in allen Zellen universell verwendbar. Zu dieser Gruppe zählen unter anderem das Adenosin-Deaminase-Gen (Kaufman et al, J. Biol. Chem. 261:9622, 1986), die Antibiotikaresistenzgene, wie zum Beispiel das Neomycin-Phosphotransferase-Gen (Southern und Berg, J. Mol. Appl. Genet. 1:327, 1982) und das Hygromycin B Phosphotransferase-Gen (hph; Blochinger and Diggelmann, Mol. Cell. Biol. 4:2929, 1984).

Obwohl hauptsächlich als rezessiver Selektionsmarker in dhfr-defizienten Zellen eingesetzt, gibt es Möglichkeiten, das dhfr-Gen unter bestimmten Voraussetzungen auch in Zellen mit endogener dhfr-Aktivität zu nutzen. Zum Beispiel vermögen transfizierte Zellen durch Verwendung eines starken Promotors für die Transkription des endogenen dhfr-Gens in moderaten Methotrexatkonzentrationen zu wachsen. Die MTX-Konzentration muß in diesem Fall höher sein als die MTX-Konzentration, die von dem endogenen dhfr-Gen kompensiert werden kann. Bei dieser Methode muß man allerdings viele falsch positive Zellklone in Kauf nehmen.

Weiters gibt es die Möglichkeit, ein mutiertes dhfr-Gen als dominanten Selektionsmarker einzusetzen (Simonsen und Levinson, PNAS 80: 2495; 1983, McIvor und Simonsen, NAR 18, 7025 ff., 1990). Diese mutierten dhfr-Gene haben eine deutlich geringere Affinität zu MTX und es ist daher möglich, höhere MTX-Konzentrationen einzusetzen, als notwendig sind, um die endogene Dihydrofolat-Reduktase zu inaktivieren.

Eine andere Möglichkeit stellt die Cotransfektion des dhfr-Gens mit einem zusätzlichen dominanten Selektionsmarker - z.B. dem Neomycin Phosphotransferase-Gen für die Resistenz gegen Geneticin (Southern, supra) -, das anschließende Überführen der Geneticin-resistenten transfizierten Zellen in Methotrexat-haltiges Medium dar (Kim and Wold, Cell 42: 129, 1985). Nach einer Cotransfektion werden allerdings oft falsch positive Klone identifiziert, die nur das dominante Selektionsmarkerplasmid aufgenommen haben.

Durch erhöhten Selektionsdruck kann man eine Amplifikation des Resistenzgens und der angrenzenden Gene beobachten. Das dhfr-Wildtyp-Gen kann mit steigenden MTX-Konzentrationen über viele Runden steigenden Amplifikationsdruckes 1000fach und mehr amplifiziert werden, während amplifizierbare dominante Marker, wie das mutierte dhfr-Gen oder das Adenosin Deaminase-Gen, nur begrenzt, zwei bis drei Schritte, amplifiziert werden können. Durch Hygromycin B-Konzentrationssteigerung konnte bisher keine deutliche Amplifikation beobachtet werden (Wirth und Hauser, "Genetic Engineering of animal cells" in "Genetic Engineering of Animals", Hrsg. Pühler, Verlag Chemie Weinheim, (1993), 1-82; Kaufman, Methods in Enzymology, Bd. 185, (1990), 537-566).

Das System dhfr-Selektion/MTX-Amplifikation stellt daher den am häufigsten verwendeten Ansatz zur Etablierung hoch-exprimierender Zellinien unter Anwendung der Coexpression heterologer Gene dar.

Auf Grund seiner rezessiven Wirkungsweise wird seine Verwendung jedoch weitgehend auf dhfr-defiziente CHO-Zellen beschränkt.

Erste Ansätze zur Coexpression und Coamplifikation von dhfr und einem Fremdgen wurden durch Cotransfektion von zwei Plasmiden gemacht. Die Plasmide werden dabei in dhfr-defiziente Zellen transfiziert. Eine Cotransfektion bringt allerdings den Nachteil mit sich, daß ein Teil der transfizierten Zellen durch die Selektion nur das dhfr-enthaltende Plasmid und nicht auch das zweite Plasmid aufnimmt.

Eine Verbesserung der Coexpression erreicht man, wenn das Markergen und das Fremdgen auf einem Plasmid angeordnet werden. Mit dieser Methode wurden unter anderem humanes Interferon β (McCormick et al., Mol. Cell Biol. 4:166, 1984), humanes Interferon γ (Haynes und Weissman, Nucl. Acids Res. 11:687, 1983;) und humanes Interleukin 2 (Onomichi, J. Biochem. 102:123, 1987) exprimiert. Die Autoren verwendeten Plasmide, in denen das dhfr-Gen und das Strukturgen jeweils einen eigenen Promotor haben. Als Expressionszellinie verwenden die Autoren eine dhfr-defiziente Hamsterzellinie CHO.

Die Abkopplung von der dhfr-defizienten Zellinie CHO zur Amplifikation und Expression von Fremdproteinen durch Verwendung mutierter dhfr-Gene wurde von Simonsen et al. und McIvor et al. (supra) versucht. Da aber die mutierten dhfr-Gene schon von vornherein wesentlich höhere MTX-Konzentrationen tolerieren, lassen sie sich nicht mehr über so viele Schritte amplifizieren, verglichen mit dem MTX-sensitiven Wildtyp dhfr-Gen.

Ein anderer Weg, um das Spektrum der möglichen Expressionszelllinien zu erweitern, wurde von Walls et al. eingeschlagen (Gene 81:139; 1989). Hier wurden Plasmide verwendet, in denen außer dem rezessiven Amplifikationsmarker dhfr auch der dominante Selektionsmarker Hygromycin B Phosphotransferase vorhanden ist. Die beiden Markergene und das Fremdgen, Protein C, bilden jeweils eine eigene Transkriptionseinheit, wobei jedes dieser Gene unter der Kontrolle eines separaten Promotors steht. In diesem multicistronischen Expressionssystem wird nur ein einziger Klon erhalten, der nach Hygromycin B (Hy B)-Selektion und anschliessender dhfr-Amplifikation auch vermehrt rekombinantes Protein C exprimiert. Andere Klone sind zwar auf Hy B selektierbar, nicht aber dhfr-amplifizierbar.

Da alle Systeme, die das Wildtyp dhfr-Gen verwenden, in der Regel auf dhfr-defiziente Zellen beschränkt sind, haben Wernicke und Will (Anal. Biochem. 203:146, 1992) eine Cotransfektion von drei Plasmiden, die jeweils das dhfr-Gen, einen dominanten Marker und das Fremdproteingen enthalten, vorgeschlagen. Sie stellen allerdings fest, daß das Fremdgen (humaner Plasminogen-Aktivator) durch Einsatz von zwei Markern nicht vermehrt exprimiert wird.

Eine weitere Verbesserung des Expressionssystems wird durch eine noch nähere Kopplung der beiden Gene, dhfr und Fremdgen, versucht. Die beiden Gene werden in ein Plasmid unter der Kontrolle von nur einem Promotor gestellt, wobei auf der gebildeten mRNA das Fremdgen gefolgt von dem Markergen als dicistronische RNA zu finden sind.

Gemäß der EP-B1 0 247 145 werden Vektoren beschrieben, in denen entweder ein Markergen und ein Gen für ein beliebiges Fremdprotein oder mindestens zwei Markergene und ein Gen für ein Fremdprotein in eine dicistronische mRNA transkribiert werden. Vergleicht man die Translationseffizienz zweier offener Leserahmen (ORF) in dicistronischen RNAs in solchen Konstrukten, so stellt man fest, daß die Translationsinitiation des stromabwärts gelegenen ORFs um das ca. 100fache ineffizienter verläuft als am stromaufwärts lokalisierten AUG des ersten ORFs (Kaufman et al., EMBO J. 6:187, 1987; Kozak, Mol. Cell. Biol. 7:3438, 1987). Der stromaufwärts liegende ORF bzw. der für die Zelle nicht essentielle ORF (Fremdgen) kann hierbei schnell durch Deletion und DNA-Rearrange-ments verloren gehen. In den Beispielen der EP-B1 0 247 145 wird auch nur die theoretische Expression eines Fremdgens in CHO-Zellen beschrieben, allerdings fehlen die Expressionsdaten. Durch die Klonierung von einem dominanten Markergen zusätzlich zum dhfr-Gen wurde versucht, das Spektrum der möglichen Expressionszellinien über die dhfr-defizienten CHO-Zellen hinaus zu erweitern. Die Chance, einen Klon, der alle drei Gene beinhält, zu erhalten, ist allerdings auf Grund der oben diskutierten Deletions- und DNA-Rearrangementsphänomene äußerst gering.

Um die Kopplung des Markergens mit dem Fremdprotein beizubehalten, aber andererseits Rearrangements und Deletionen zu reduzieren, wurde versucht, zwischen die dicistronischen Leserahmen Sequenzelemente, an denen Ribosomen intern zu binden vermögen, einzubringen. Diese Sequenzelemente werden "Internal Ribosome Entry Sites" (IRES) genannt, und wurden erstmals in der Familie der Picornaviren gefunden. Die 5'-untranslatierten Regionen (UTR) von Poliovirus (Pelletier and Sonenberg, Nature 334:320, 1988) und Encephalomyocarditis (EMC) Virus (Jang et al., J. Virol. 63:1651; 1989;) sind in der Lage, in Zellen die interne Bindung der Ribosomen und damit verbunden die Translationsinitiation an mRNAs zu vermitteln. Durch Insertion dieser Sequenz zwischen die beiden offenen Leserahmen (Fremdprotein und Selektionsmarker) erreicht man eine gekoppelte, und dadurch effizientere Translation auch des stromabwärts liegenden Leserahmens in der dicistronischen Einheit (Jang, supra) und vermeidet so Rearrangements und Deletionen (Kaufman, Nucl. Acids Res. 19:4485; 1991). In tricistronischen Konstruktionen, in denen dem dritten Cistron die IRES-Sequenz vorgeschaltet ist, wird zumindest der zweite ORF deletiert. Ist hingegen dem zweiten Cistron die IRES vorgeschaltet, so wird der dritte ORF nur mäßig bis gar nicht translatiert. Er unterliegt den Gesetzen, wie sie für dicistronische Konstruktionen ohne IRES gelten (Jang, supra).

Gemäß der DE-A 42 28 458 wird dieses System benutzt, um eine multicistronische Expressionseinheit zu konstruieren, welche die äquimolare Expression der in den jeweiligen Cistrons positionierten Gene erlaubt. Stromabwärts der IRES Sequenz ist eine Nukleotidsequenz 'Y' eingefügt, die die geforderte äquimolare Expression der Fremdgene bewirken soll. Diese Expressionseinheiten sind insbesondere zur Herstellung von rekombinanten Proteinen geeignet, die aus zwei oder mehreren Proteinuntereinheiten bestehen. Als Beispiel für solche rekombinanten Proteine wird das Gen für den "Platelet Derived Growth Factor", der aus einer A- und B-Kette besteht, mit diesem System exprimiert.

Die Verwendung eines Fusionsproteins aus zwei dominanten Selektionsmarkern wird in der WO 92/08796 beschrieben. Hierbei wird ein positiv selektierbares Gen (Hygromycin B-Phosphotransferase, hph) und ein negativ selektierbares Gen (Thymidinkinase des Herpes Simplex Viruses, HSV-1 TK) so fusioniert, daß dem entstehenden Fusionsprotein der C-Terminus des Hygromycin B-Proteins und der N-Terminus des HSV-1 TK-Proteins fehlt. Es wird gezeigt, daß das Fusionsprotein bifunktionell aktiv ist und eine dieses Gen exprimierende Wirtszelle einen dominant positiv selektierbaren und negativ selektierbaren Phenotyp bekommt.

Ein ebenfalls bifunktionelles Fusionsprotein konstruierten Schwartz et al. (PNAS 88:10416, 1991). Die Autoren fusionierten das HSV-1 TK-Gen mit dem bakteriellen Neomycin-Phosphotransferase (neo)-Gen in der Art, daß das am C-Terminus modifizierte HSV-1 TK-Gen an das Startkodon des neo-Gens im Leserahmen ligiert wurde.

Alle bisher beschriebenen Strategien zur Optimierung der Expression wurden erarbeitet, um Fremdproteine in großem Maßstab herzustellen. Zum Beispiel braucht man zur Herstellung von rekombinanten Impfstoffen eine große Menge gereinigter Proteine. Für die Behandlung von Patienten mit Defekten in der Blutgerinnung ist die Verfügbarkeit von großen Kontingenten an Plasmaproteinen von immenser Bedeutung.

Prothrombin konnte von Jorgensen et al. (J. Biol Chem. 262: 6729, 1987) in CHO-Zellen ohne Amplifikation in einer Konzentration von 100 ng Prothrombin/10⁶ Zellen in 24 h exprimiert werden. Nach Amplifikation über dhfr waren die Ausbeuten bei 8-11 mU Prothrombin/10⁶-Zellen in 24 h. Durch Expression von Prothrombin mit dem Vaccinia Virus System konnte eine Expression von 18-23 mU/10⁶-Zellen und Tag erreicht werden (Falkner et al., Throm. and Haem. 68:119, 1992).

Die cDNA für den humanen Faktor VIII kodiert für 2332 Aminosäuren. Im Plasma liegt allerdings nur ein Bruchteil von Faktor VIII als einkettiges Protein vor. Die vorherrschende Fäktor VIII-Spezies ist ein Zweikettenmolekül, bestehend aus einer leichten und einer unterschiedlich langen schweren Kette. Erste Versuche, rekombinanten Faktor VIII zu exprimieren, stellten sich als schwierig heraus, da die Prozessierung eines so kompliziert aufgebauten Proteins in Wirtszellen sehr ineffizient durchgeführt wird. Kaufman et al. (J. Biol. Chem. 263:6352, 1988) konnten in hoch-amplifizierten CHO-Zellen (20µM bzw. 1mM MTX) maximal 1U FVIIIc/10⁶-Zellen in 24 Stunden exprimieren. Dieser Wert kam nach 10000-facher Expressionssteigerung zustande. Anfangs lag die FVIIIc-Expression nur am Rande der Nachweisgrenze.

Mehrere Ansätze haben dann gezeigt, daß ein rekombinantes Faktor VIII-Protein, dem ein großer Teil der schweren Kette fehlt, ebenfalls koagulative Eigenschaften besitzt, die vom nativen Molekül nicht zu unterscheiden sind (Eaton et al., Biochemistry 25:8343, 1986; Mertens et al., Brit. J. Haematol. 85:133, 1993). Auch in vivo wird dem Faktor VIII durch Prozessierung die B-Domäne abgespalten. Mehrere Autorengruppen konnten sogar zeigen, daß die Expression von B-Domänen-deletiertem Faktor VIII wesentlich besser funktioniert als die Expression der kompletten Faktor VIII cDNA (Toole et al. PNAS 83:5939; 1986; Pittman et al., Blood 81:2925, 1993). Diese Literaturstellen geben 10-20fach höhere Expression von deletiertem FVIII gegenüber FVIIIc an. Diese Expressionswerte konnten jedoch erst nach Amplifikation auf 1µM bzw. 5µM MTX und vWF-Coexpression erreicht werden.

Gemäß der US-A 5 171 844 konnte die Faktor VIII-Deletionsmutante FVIIIdB928 in COS-Zellen transient in einer Konzentration von 15 mU/ml in 48 h Kultur exprimiert werden.

Gemäß der EP-A 0 351 586 wird ein Expressionsplasmid mit einem Faktor VIII, dem die Aminosäuren 740 bis 1649 fehlen, unter der Kontrolle des Hühner β-Actin-Promotors beschrieben. Wird dieses Plasmid mit einem zweiten, dhfr-exprimierenden Plasmid in CHO-Zellen kotransfiziert und anschließend mit 10 nM MTX amplifiziert, kann man die Expression von FVIII:C von ca. 350 mU/10⁶ Zellen pro Tag auf 1300 mU/10⁶ Zellen pro Tag erhöhen. Im Vergleich zu dieser Kotransfektion zeigt die Transfektion mit einem Plasmid, in dem sich sowohl das dhfr-Gen unter Kontrolle des SV40-Promotors als auch die cDNA des deletierten Faktors VIII unter der Kontrolle des Hühner β-Actin-Promotors befinden, eine weit geringere Initialexpression des Faktors VIII, wie das nicht amplifizierte monocistronische Plasmid.

Der humane Faktor IX wurde in dhfr-defizienten CHO-Zellen mit einem Plasmid exprimiert, welches die Faktor IX-cDNA und das dhfr-Gen unter der Kontrolle des Adenovirus major late-Promotors exprimiert (Kaufman et al., J. Biol. Chem. 261:9622, 1986). Doch selbst bei Amplifikation mit 20 µM MTX wurden bei bis zu 188,0 µg/ml erhaltenem Faktor IX nur 0,2 bis 4,4% funktioneller Faktor IX produziert. Das von Balland et al. beschriebene CHO-Expressionssystem erreicht bei etwa 2µg Faktor IX/ml und 24 Stunden nur etwa 30% funktionellen Faktor IX (Eur. J. Biochem. 172: 565, 1988). In der WO 86/06408 wird außerdem beschrieben, dass von nicht amplifizierten CHO-Zellen nur 15ng Faktor IX/ml und 24 Stunden produziert werden.

Protein C wird von Grinell et al. (Adv. Appl. Biotechnol. Series 11:29, 1990) in initialselektierten, nicht amplifizierten Zellklonen in einer maximalen Menge von 1,15 µg/10⁶-Zellen und Tag exprimiert. Gemäß der US 4 775 624 werden 1,8 µg/ml Protein C in CHO DUKX B11-Zellen exprimiert. Auch in der EP-B1 0 266 190 wird eine Protein C-Expression von 1-2 µg/10⁶-Zellen in BHK und 293 Zellen dokumentiert.

Die vorliegende Erfindung stellt sich daher die Aufgabe, ein System zur Verfügung zu stellen, das eine Expression eines Fremdproteins in hoher Ausbeute und Reinheit ermöglicht.

Diese Aufgabe wird erfindungsgemäß gelöst durch ein Expressionsplasmid, das eine dicistronische Transkriptions-/Translationseinheit enthält, welche eine Sequenz für ein Fremdprotein, eine interne Ribosomenbindungsstelle und eine Sequenz für ein Fusionsprotein umfasst, wobei das Fusionsprotein aus mindestens einem Amplifikationsmarkerprotein und mindestens einem Selektionsmarkerprotein besteht, wobei der Amplifikationsmarker die Dihydrofolatreduktase ist. Die erfindungsgemäßen Expressionsplasmide ermöglichen bei der Expression von Fremdproteinen in dafür geeigneten eukaryontischen Zellen einerseits ein sehr hohes Verhältnis von Fremdprotein exprimierender Klonen zu den Gesamtklonen und andererseits eine überraschend hohe Initialexpression der Fremdproteine.

Die interne Ribosomenbindungsstelle gewährleistet eine zuverlässigere Translation der gesamten mRNA.

Eine besonders bevorzugte interne Ribosomenbindungsstelle ist die 5'-nicht-translatierte Region des Encephalomyocarditis-Virus (ECMV 5'UTR). Diese ermöglicht eine besonders gute Bindung der Ribosomen im internen Bereich der mRNA, wodurch die Translation eines weiter stromabwärts liegenden offenen Leserahmens positiv beeinflusst wird.

Gemäß einer bevorzugten Ausführungsform der erfindungsgemäßen Plasmide liegt die kodierende Sequenz für das Fremdprotein 5' und die kodierende Sequenz für das Fusionsprotein 3' von der internen Ribosomenbindungsstelle. Diese Anordnung ermöglicht eine maximale Ausbeute an Fremdprotein, da das Gen für das Fremdprotein unmittelbar nach dem Promotor liegt und damit optimal transkribiert wird.

Das Fremdgen und die Sequenz für das Fusionsprotein sind vorzugsweise in eine dicistronische mRNA transkribierbar, weil dadurch die Transkription/Translation in einfachster Weise ablaufen kann.

Die erfindungsgemäßen Expressionsplasmide werden bevorzugt von einem einzigen, möglichst starken Promotor kontrolliert, beispielsweise durch den CMV-, den SV40-, den humanen β-Actin- oder vergleichbare Promotoren.

Zusätzlich können die erfindungsgemäßen Plasmide ein Intron, vorzugsweise das Intron des SV40 t-Antigens, das 16s/19s-Intron oder das erste Intron des humanen β-Actin-Gens, und/oder ein Polyadenylierungssignal, vorzugsweise das der frühen oder späten Transkriptionseinheit des SV40-Virus, enthalten. Auch diese Bestandteile ermöglichen optimierte Expressionsraten des Fremdproteins.

Gemäß einer bevorzugten Ausführungsform des erfindungsgemäßen Plasmids besteht die Sequenz für das Fusionsprotein aus zwei Teilsequenzen, nämlich einem hochamplifizierbaren Amplifikationsmarkergen, dem Dihydrofolat Reduktase-Gen, und einem Selektionsmarkergen, vorzugsweise dem Hygromycin B-Phosphotransferase-Gen.

Das Dihydrofolat Reduktase-Gen/Hygromycin B-Phosphotransferase-Gen-System hat den besonderen Vorteil, dass dieses Fusionsprotein durch die enge Kopplung der hph- und dhfr-Domänen als dominanter Marker auch in Zellen mit endogenem dhfr-Gen amplifiziert werden kann. Dies wird insbesondere auch durch die Eigenschaft eines hph-Amplifikationspotentials ermöglicht, sodaß man von einem doppelt dominanten selektierbaren und doppelt amplifizierbaren Markerprotein sprechen kann. So kann man zunächst eine genügend hohe hph-Amplifikation durchführen, die bei anschließendem Umschalten auf MTX gewährleistet, daß die dann gewählte MTX-Konzentration nicht mehr vom endogenen DHFR kompensiert werden kann.

Vorzugsweise ist das Selektions-/Amplifikationsmarker-Fusionsprotein bifunktionell und die für das Fusionsprotein kodierende Sequenz so konstruiert, daß der 5'-kodierenden Teilsequenz das Stopkodon und der 3'-kodierenden Teilsequenz gegebenenfalls das Startkodon fehlt. Dadurch kann das Fusionsprotein in einfacher und effizienter Weise translatiert werden.

Bei einer anderen Ausführungsart des Expressionsplasmids sind die kodierenden Sequenzen der beiden Proteinanteile der Sequenz für das Fusionsprotein durch einen Spacer getrennt, insbesondere durch einen 15 Nukleotide langen Spacer. Vorzugsweise kodiert die Spacersequenz für 5 Glycinreste (GGA GGC GGG GGT GGA (SEQ.ID.NO. 2)) oder für 5 Prolinreste und die Sequenz CCA CCC CCG CCT CCA (SEQ.ID.NO. 1).

Das Vorhandensein des Spacerproteins fördert die Funktionalität des Fusionsprotein. Die Aktivität der Markerproteine im Fusionsprotein gegenüber den distinkten Markerproteinen ist nicht verringert.

Die Aminosäuresequenzen von bevorzugten Fusionsproteinen sind als SEQ.ID.NO.3 (Fusionsprotein DHFR/HPH ohne Spacer), SEQ.ID.NO.4 (Fusionsprotein DHFR/HPH mit Glycin-Spacer) und SEQ.ID.NO.5 (Fusionsprotein DHFR/HPH mit Prolin-Spacer) im Sequenzprotokoll aufgelistet.

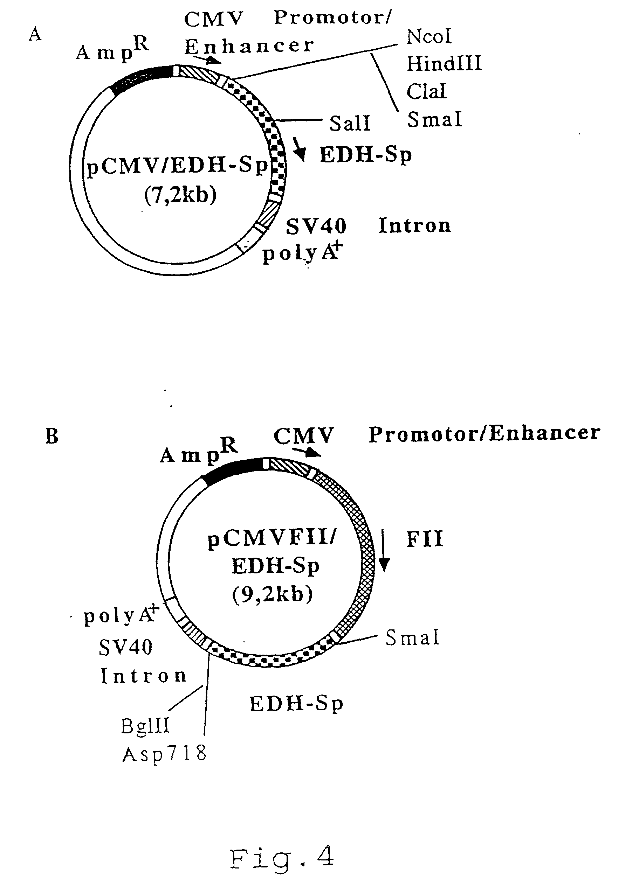

Beispiele für bevorzugte Plasmide sind die Expressionsplasmide pCMV/EDH-Sp, pCMV/EDHGly und pCMV/EDHPro gemäß Fig.4-A.

Die erfindungsgemäßen Expressionsplasmide eignen sich in besonderer Weise für die Expression von humanen Plasmaproteinen oder viralen Proteinen, bzw. deren Derivate oder Fragmente.

Bevorzugte Proteine, die mit den erfindungsgemäßen Plasmiden exprimiert werden können, sind humanes Prothrombin, humaner Faktor VIII, insbesondere die Deletionsmutante FaktorVIIIdB928 des Faktor VIII, welche die größte Deletion in der B-Domänen aufweist, die noch die Expression eines aktiven Faktor VIII zulässt, humaner Faktor IX, humanes Protein C, humanes Serumalbumin (HSA) und humaner von Willebrand-Faktor.

Bevorzugte Expressionsplasmide sind
- pCMVFII/EDH-Sp, pCMVFII/EDHGly und pCMVFII/EDHPro (zur Expression von Prothrombin),
- pCMVFVIIIc/EDH-Sp, pCMVFVIIIc/EDHGly und pCMVFVIIIC/EDHPro (zur Expression von Faktor VIII),
- pCMVFVIIIdB928/EDH-Sp, pCMVFVIIIdB928/EDHGly, pCMVFVIIIdB928/EDHPro (zur Expression von FVIIIdB928),
- pCMV-FIX-EDH-Sp, pCMV-FIX-EDHGly und pCMV-FIX-EDHPro (zur Expression von Faktor IX),
- pCMV-PCwt-EDH-Sp, pCMV-PCwt-EDHPro, pCMV-PCwt-EDHGly, pCMV-PCpt. mut.-EDH-Sp, pCMV-PCpt.mut.-EDHPro und pCMV-PCpt. mut.-EDHGly (zur Expression von Protein C),
- pAct-vWF-EDH-Sp, pAct-vWF-EDHPro und pAct-vWF-EDHGly (zur Expression von von Willebrand-Faktor.

Als besonders vorteilhaft haben sich Expressionsplasmide gezeigt, welche Expressionskassetten umfassen, die die DNA Sequenzen SEQ.ID.NO. 6, SEQ.ID.NO. 7 oder SEQ.ID.NO. 8 enthalten, und eine hervorragende Expression insbesondere des Fremdproteins in der transfizierten Zelle ermöglichen.

Das im Rahmen der vorliegenden Erfindung verwendete Fusionsprotein besteht aus einem hochamplifizierbaren Amplifikationsmarker (DHFR) und einem Selektionsmarker.

Dieses Fusionsprotein ist bevorzugt dadurch gekennzeichnet, dass dem 5'-kodierenden Gen für den Amplifikationsmarker das Stop-kodon und dem 3'-kodierenden Gen für den Selektionsmarker das Startkodon fehlt.

Bei einem weiteren bevorzugten Fusionsprotein sind der Amplifikationsmarker und der Selektionsmarker durch ein Spacerprotein getrennt, das vorzugsweise aus mindestens 5 Glycinresten oder aus mindestens 5 Prolinresten besteht.

Beispiele für solche bevorzugten Fusionsproteine weisen die Aminosäuresequenz SEQ.ID.NO. 3, SEQ.ID.NO. 4 oder SEQ.ID.NO. 5 auf.

Ein weiterer Aspekt der Erfindung betrifft transfizierte eukaryontische Zelllinien, vorzugsweise ausgewählt aus den Zelllinien CHO, 293 oder humane Leberzelllinien, wie SK-HEP-1 oder Chang Liver, die mit einem erfindungsgemäßen Expressionsplasmid transfiziert sind und ein Fremdprotein exprimieren.

Gemäß einem anderen Aspekt der Erfindung setzt man die Zelllinie SK-Hep-1 als Expressionsvehikel, insbesondere für humane Plasmaproteine, wie Prothrombin, Faktor VIII (bzw. Faktor VIII-Derivate, wie die Mutante Faktor VIII dB928), Faktor IX, Protein C oder von Willebrand Faktor, ein.

Vorzugsweise exprimiert die transfizierte eukaryontische Zelllinie humanes Prothrombin, humanen Faktor VIII, die Deletionsmutante dB928 des humanen Faktor VIII, den humanen Faktor IX, das humane Protein C, humanes Serumalbumin (HSA) oder den humanen von Willebrand Faktor, bzw. deren Derivate oder Fragmente.

Die Erfindung betrifft auch ein Verfahren zur Herstellung von Fremdproteinen, welches dadurch gekennzeichnet ist, dass eine eukaryontische Zelllinie mit einem erfindungsgemäßen Expressionsplasmid transfiziert wird, die erhaltenen Klone durch einen Selektionsprozeß unter der Kontrolle des Selektionsmarkers isoliert und dabei vorzugsweise gleichzeitig amplifiziert werden, anschließend unter der Kontrolle eines Amplifikationsmarkers weitere Amplifikationen erfolgen, wobei das Fremdprotein exprimiert und geerntet wird.

Bei einer bevorzugten Verfahrensvariante erfolgt der Selektionsprozeß unter Verwendung von Hygromycin B und die weitere Amplifikation unter Verwendung von Methotrexat.

Dabei hat es sich gezeigt, daß einerseits die Kombination der Amplifizierbarkeit und der dominanten Selektierbarkeit des dhfr-Gens sowie die enge Verbindung des Amplifikations-Selektionsmarkerproteingens mit dem Fremdgen in einer dicistronischen Transkriptions/Translationseinheit für die Ausbeute an Fremdprotein von großer Bedeutung ist.

Beim Optimieren des Expressionsprotokolls unter Verwendung der erfindungsgemäßen Expressionsplasmide kam es zu dem überraschenden Ergebnis, daß auch das Hygromycin B-Phosphotransferase-Gen amplifizierbar ist. Dies widerspricht der allgemeinen Auffassung. Durch langsame Steigerung der Hy B-Konzentration konnte unter anderem auch eine Koamplifikation des dhfr-Gens erreicht werden, die eine Umstellung auf eine MTX-Konzentration erlaubte, die für das endogene DHFR bereits toxisch ist. Dann erst wurde mit MTX die eigentliche Amplifikation über mehrere Schritte durchgeführt.

Diese bevorzugte Kombination des rezessiven Amplifikationsmarkers dhfr mit dem dominanten Selektionsmarker hph als Fusionsprotein erlaubt die Amplifikation der Fremdgene bzw. Expression der Fremdproteine in jeder beliebigen Zellinie. Bevorzugt werden die Zellinien, die die Prozessierung und Modifikation der Proteine vollständig durchführen.

Als besonders bevorzugte Zellinien im erfindungsgemäßen Verfahren haben sich CHO, 293 oder humane Leberzellinien, wie SK-HEP-In und Chang Liver (ATCC CCL 13) erwiesen.

In den Beispielen werden sowohl die dhfr-defiziente Zellinie CHO DUKX-B11 (Chasin und Urlaub, PNAS 77:4216, 1980), als auch Zelllinien mit endogenem dhfr-Gen, 293 (ATCC CRL 1573) und SK-HEP-1 (ATCC HTB 52) verwendet.

Erfindungsgemäß eignen sich Leberzellinien am besten für die Expression des humanen Faktors VIII. Bei Verwendung dieser Zelllinien wurde überraschenderweise festgestellt, daß nicht nur 95% der Faktor VIII transformierten Zellen auch Faktor VIII exprimieren, sondern daß auch initial schon eine große Menge an Faktor VIII exprimiert wird. Nicht zuletzt zeigen diese Leberzelllinien eine optimale post-translationale Modifikation des rekombinanten Faktors VIII.

Insbesondere zeigte sich von allen Leberzellinien die Zellinie SK-HEP-1 als besonders gut geeignet.

Erfindungsgemäß werden bevorzugt rekombinante Blutgerinnungsfaktoren, insbesondere rekombinantes humanes Prothrombin, rekombinanter humaner Faktor VIII, rekombinanter humaner FVIIIdB928, rekombinanter humaner Faktor IX, rekombinantes humanes Protein C, humanes Serumalbumin (HSA) oder rekombinanter humaner von Willebrand Faktor, hergestellt.

Schließlich betrifft die Erfindung auch Fremdprotein-Präparationen, welche durch das erfindungsgemäße Verfahren erhältlich sind und sich durch einen besonders hohen Anteil an aktivem Protein und hoher Reinheit auszeichnen, insbesondere auch bei Proteinen, die post-translationelle Modifikationsprozesse durchlaufen müssen, um in ihre aktive Form gebracht zu werden.

Daher betrifft die vorliegende Erfindung insbesondere Präparationen von viralen Proteinen oder von humanen Plasmaproteinen, vorzugsweise von aktivem humanen Prothrombin, von aktivem humanen Faktor VIII, von aktivem humanen, deletiertem FVIIIdB928, von aktivem humanen Faktor IX, von aktivem humanen Protein C, von HSA und von aktivem humanen von Willebrand-Faktor.

Die Erfindung betrifft weiters pharmazeutische Zusammensetzungen, welche eine dieser erfindungsgemäßen Präparationen, insbesondere Plasmaproteinpräparationen, umfaßt. Diese pharmazeutischen Zusammensetzungen werden durch übliche Verfahren aus den erfindungsgemäßen Präparationen erhalten und zeichnen sich durch eine besonders gute Wirksamkeit bzw. Verträglichkeit aus, welche durch das effiziente Herstellungsverfahren der Präparationen bedingt sind.

Durch die erfindungsgemäße Anordnung und Art der funktionellen Segmente (Fremdgen, Markerfusionsproteingen) im Plasmid werden einerseits Deletionen und DNA-Rearrangements verhindert, andererseits aber die Funktionalität beider Markerelemente und auch die mengenmäßig überraschend hohe Expression der diversen Fremdproteine in funktioneller Form gewährleistet. Bei allen untersuchten Fremdproteinen zeigte sich bereits eine sehr hohe Initialexpression. Zum Beispiel wird Prothrombin, wie oben erwähnt, in CHO ohne Amplifikation in einer Menge von 100 ng/10⁶ Zellen in 24h exprimiert (Jorgensen et al., supra). Im nachfolgenden Beispiel 1 wird gezeigt werden, daß mit dem erfindungsgemäßen Expressionsplasmid Prothrombin in CHO-Zellen ohne Amplifikation bereits in einer Menge von 12 bis 15 mU/10⁶ Zellen in 24h (dies entspricht 1,2 bis 1,5 µg), und in 293-Zellen sogar 50 bis 55 mU/10⁶ Zellen in 24h (entspricht 5 bis 5,5 µg) produziert werden konnte. Ebenso können die Expressionswerte, die in der Literatur für andere Plasmaproteine erst nach umfangreicher Amplifikation erreicht werden, mit dem erfindungsgemäßen Expressionsplasmid , schon im Stadium der Initialexpression drastisch überschritten werden. Besonders wird darauf hingewiesen, daß die hier angegebenen Expressionsdaten nicht die Menge an exprimiertem, antigenem Protein aufzeigen, sondern daß es sich um Proteinmengen handelt, die in Aktivitätstests ermittelt worden sind.

Die Erfindung wird anhand der Zeichnung sowie der nachstehenden Beispiele, auf die sie jedoch nicht eingeschränkt sein soll, näher erläutert. In der Zeichnung zeigen:
Fig. 1 die Anordnung des EDH-Selektions-/Amplifikationsmarkers im Kontext mit Promotor und Fremdgen, wobei der Pfeil die Richtung der Transkription anzeigt;
Fig. 2 die Konstruktion der ED-Kassette und Subklonierung in pCRTM;
Fig. 3 die Struktur der Plasmide pCMVNco/MCS (A) und pCMV/Hy (B);
Fig. 4 die Struktur der Plasmide pCMV/EDH-Sp (A) und pCMVFII/EDH-Sp (B);
Fig. 5A-C die Aminosäuresequenz der Fusionsproteine: DHFR/HPH ohne Spacer (A; SEQ.ID.NO. 3), DHFR/HPH mit Glycin-Spacer (B, SEQ.ID.IVO. 4) und DHFR/HPH mit Prolin-Spacer (C, SEQ.ID.NO. 5), wobei die Sequenz im Ein-Buchstabencode angegeben ist;
Fig. 6 die Southern Blot-Analyse von genomischer DNA der CHO-Zellklone #837 (transfiziert mit pCMVFII/EDH-Sp, DHFR Initialselektion) und #4399 (Subklon von #837, amplifiziert auf 40nM MTX);
Fig. 7 die Western Blot-Analyse von 293 bzw. CHO-Zellklonen, die mit dem Plasmid pCMVFII/EDHPro bzw. pCMVFII/EDH-Sp. transfiziert wurden;
Fig. 8 die Struktur der Plasmide pCMVFVIIIc/EDHPro (A) und pCMVFVIIIdB928/EDHPro (B);
Fig. 9 die Southern Blot-Analyse von genomischer DNA der SK-HEP-1 Zellklone #1963 (400µg HyB/ml) und #3310 (1500µg HyB/ml),wobei der Klon #3310 von #1963 abstammte;
Fig. 10 die Western Blot-Analyse von FVIIIdB928-exprimierenden 293-und SK-HEP-1-Zellen;
Fig. 11 die Struktur des Plasmides pActvWF/EDHPro;
Fig. 12 die Konstruktion von pCMV-FIX-EDHPro;
Fig. 13 ein Western Blot von rekombinantem Faktor IX aus 293- und SK-HEP-1-Zellklonen im Vergleich zu plasmatischem Faktor IX und rekombinantem Faktor IX aus CHO-Zellen.
Fig. 14 die Konstruktion von pCMV-PCwt-EDHPro und pCMV-PCpt.mut.-EDHPro;
Fig. 15 ein Western Blot von rekombinantem Protein C aus 293 und SK-HEP-1-Zellen im Vergleich zu plasmatischem Protein C;

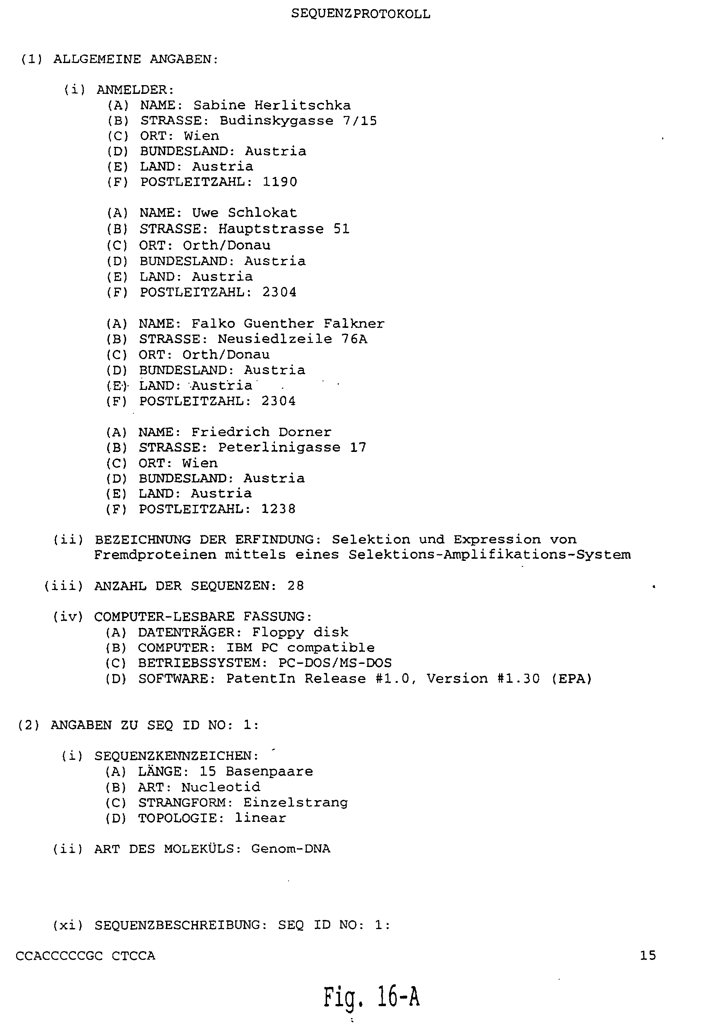

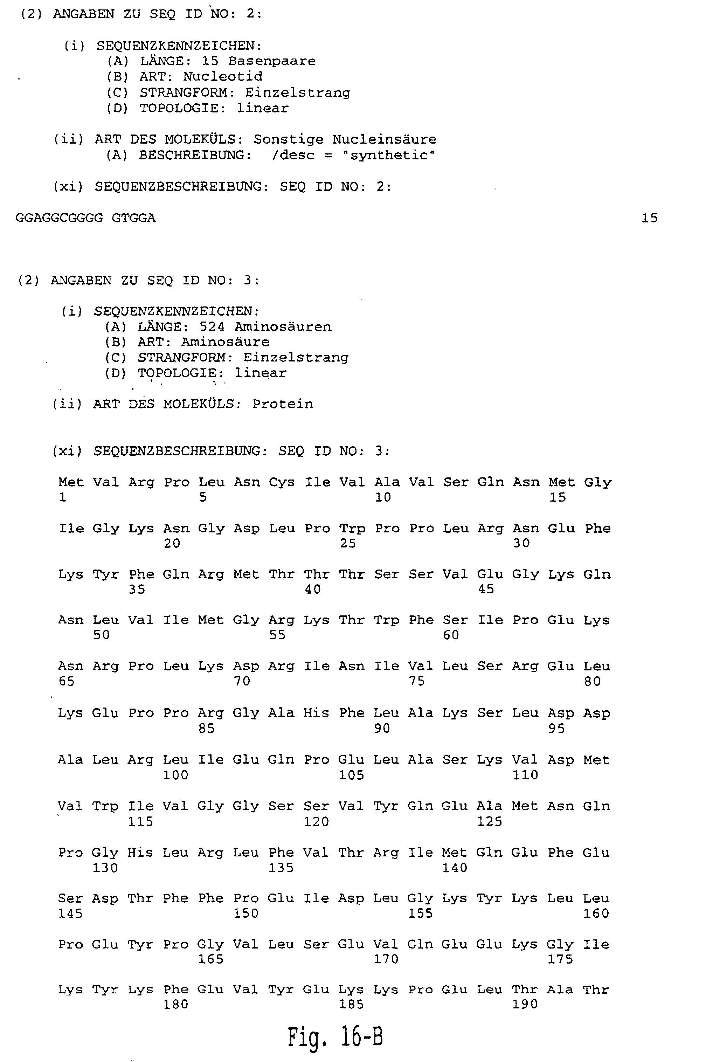

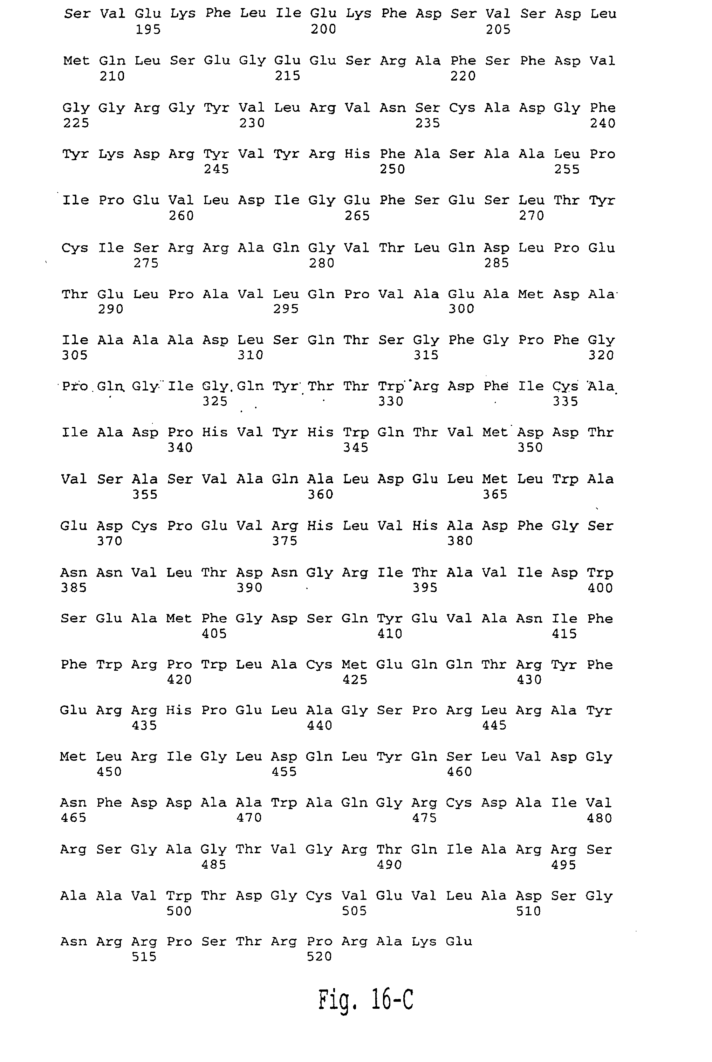

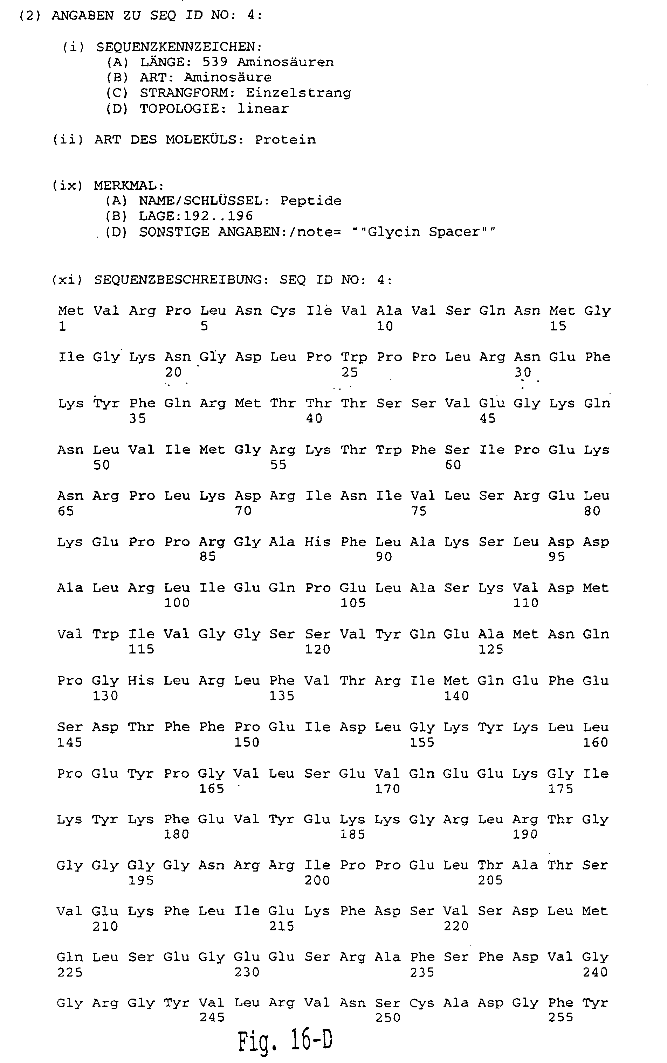

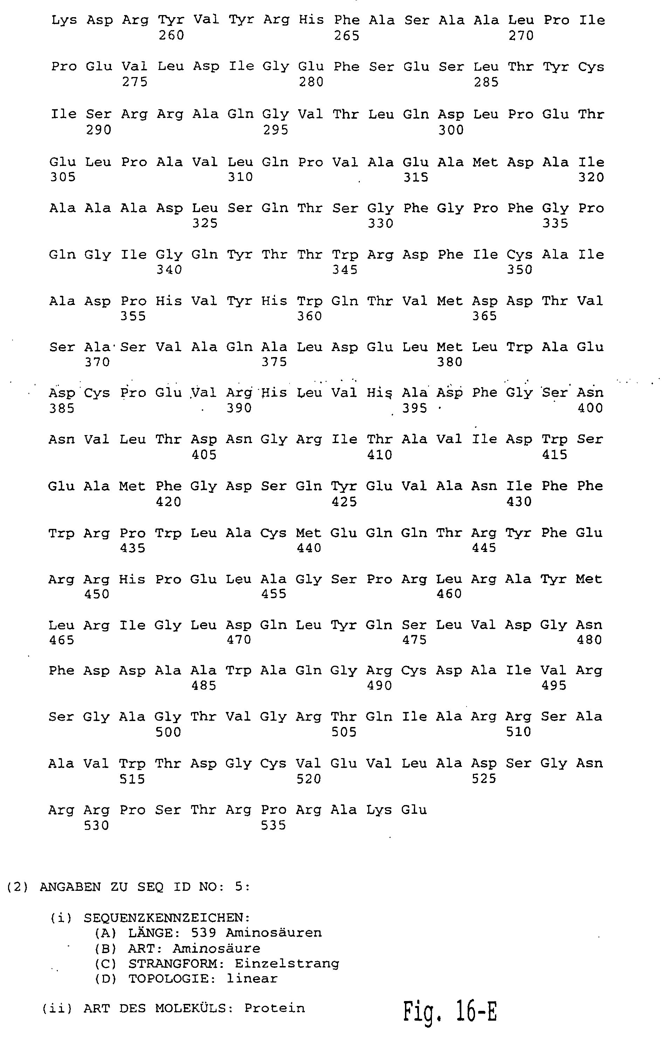

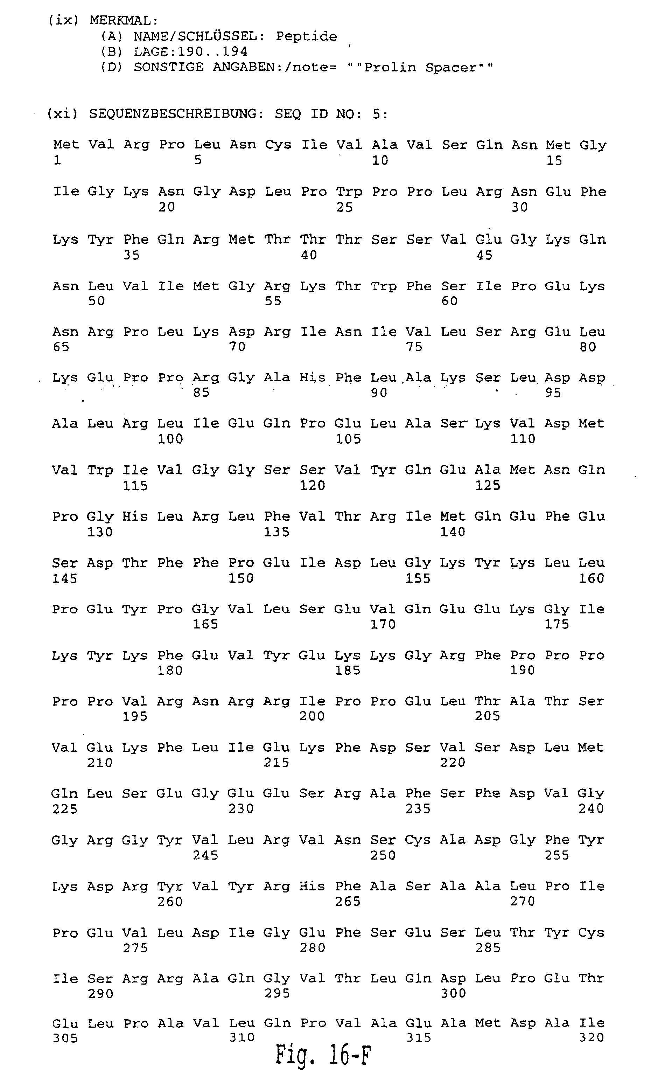

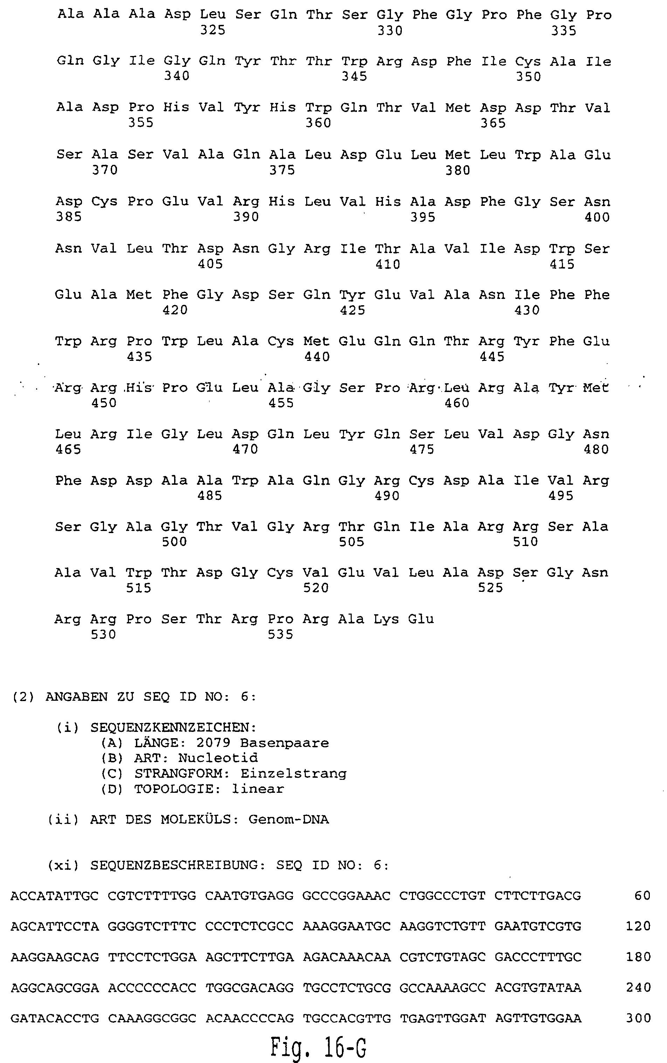

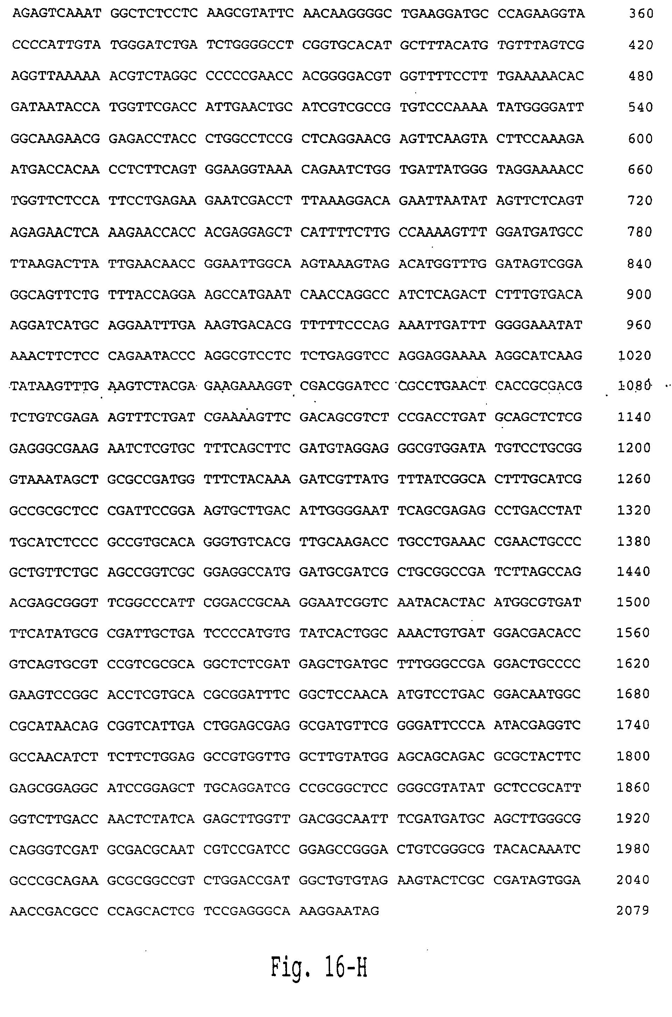

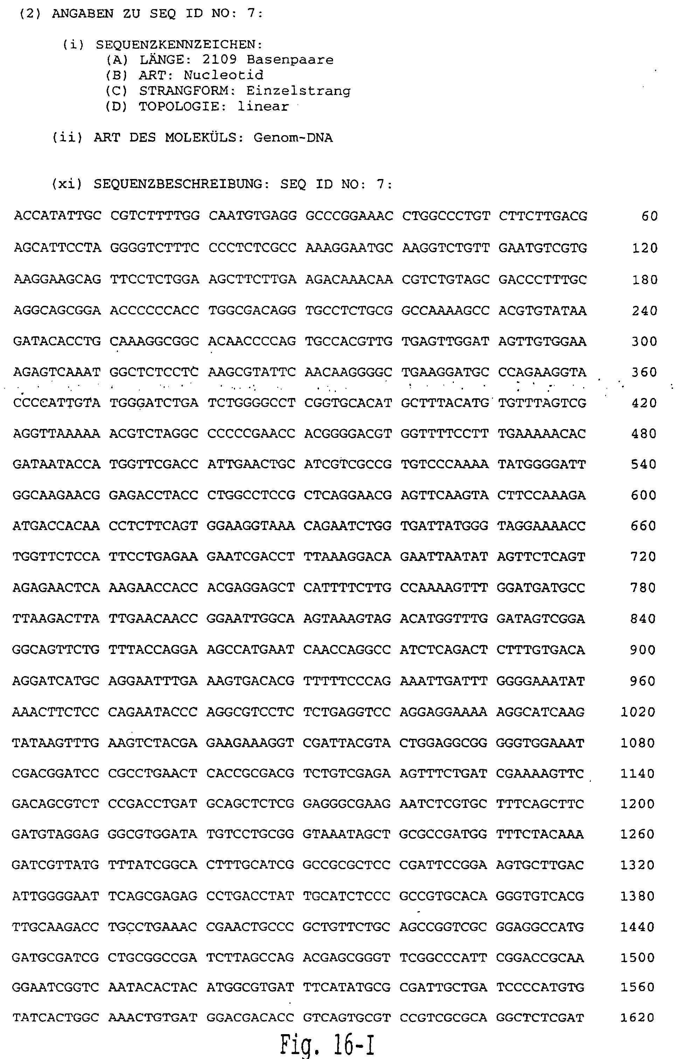

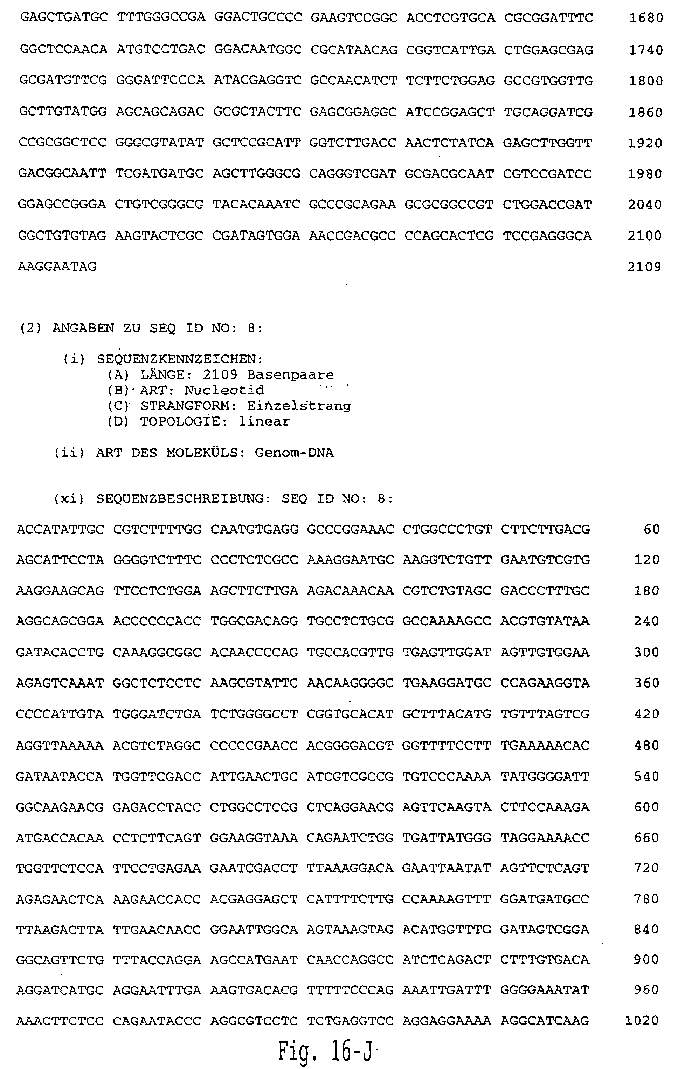

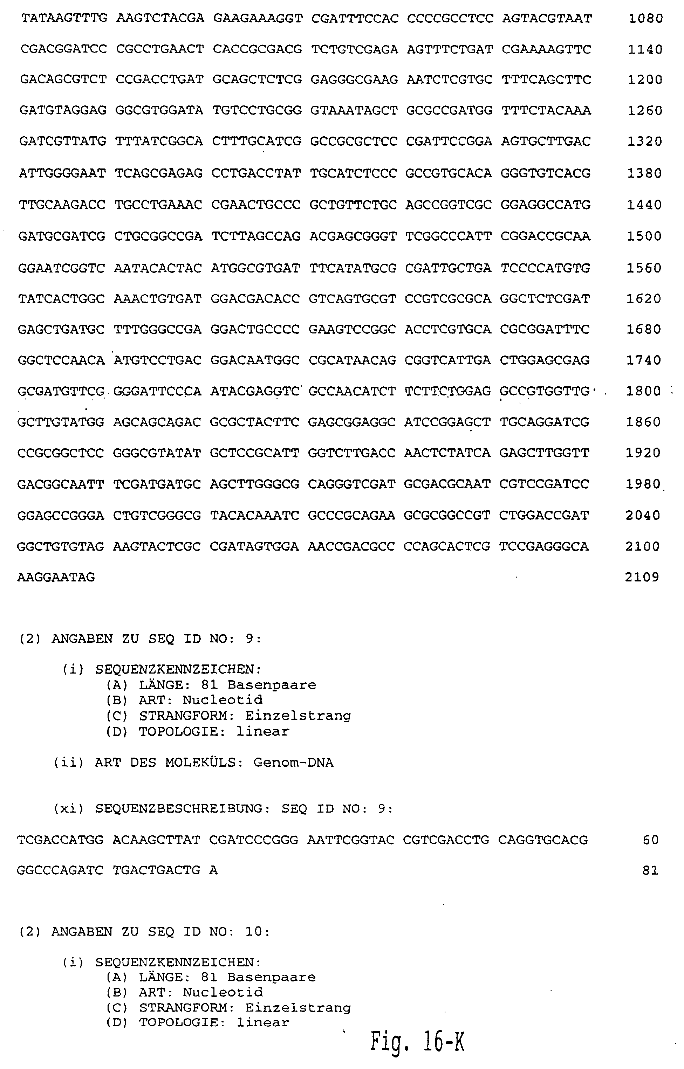

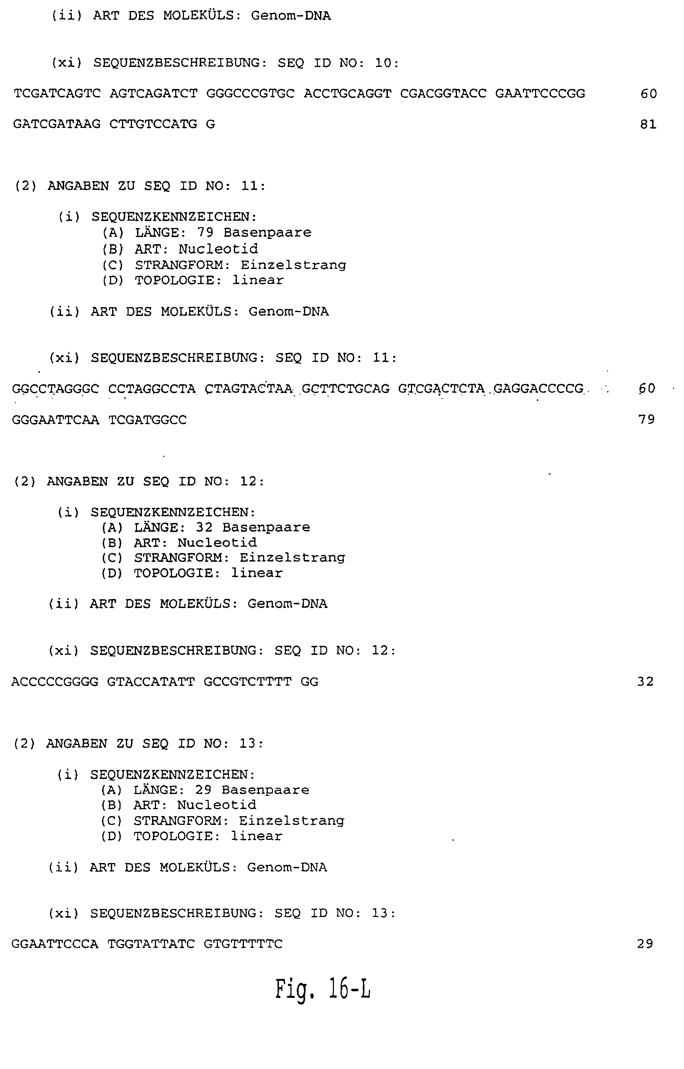

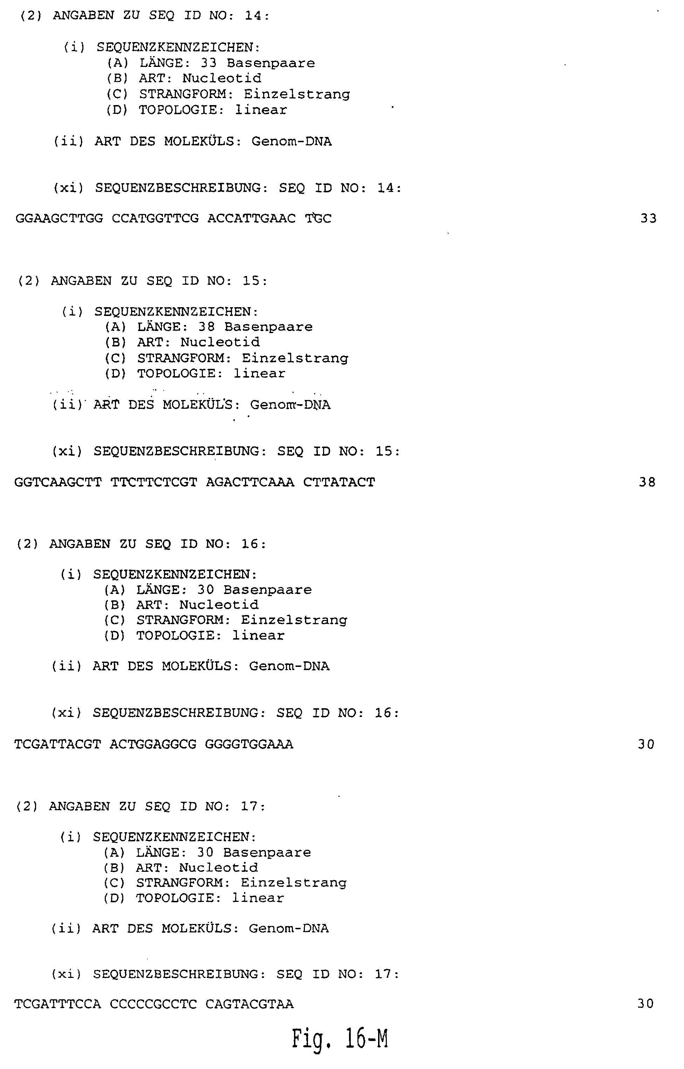

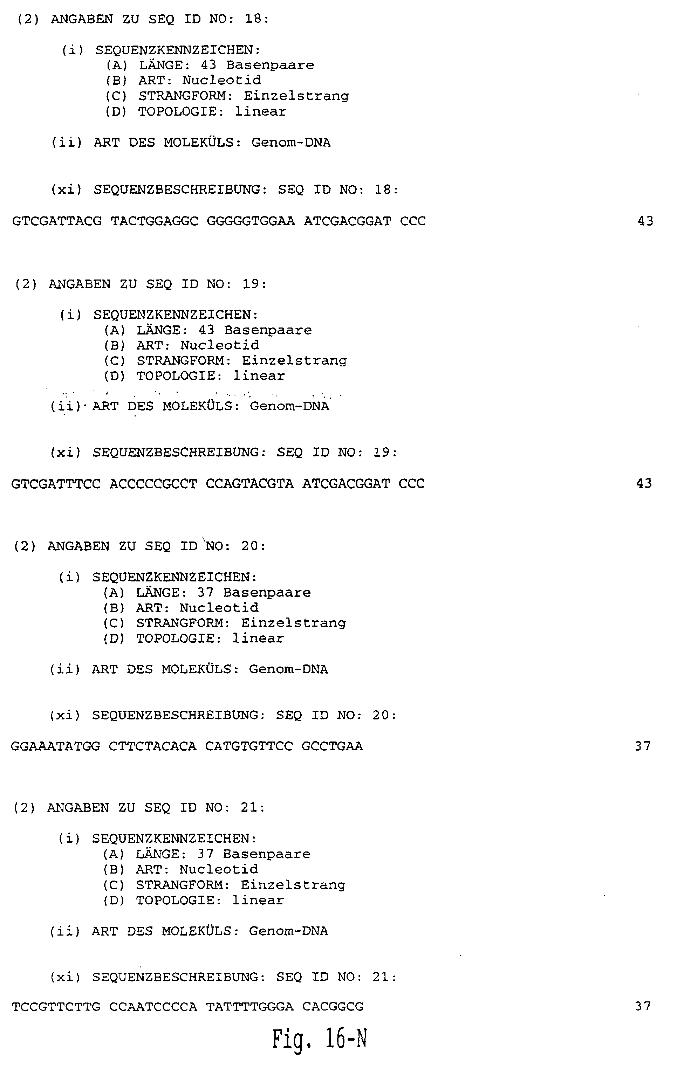

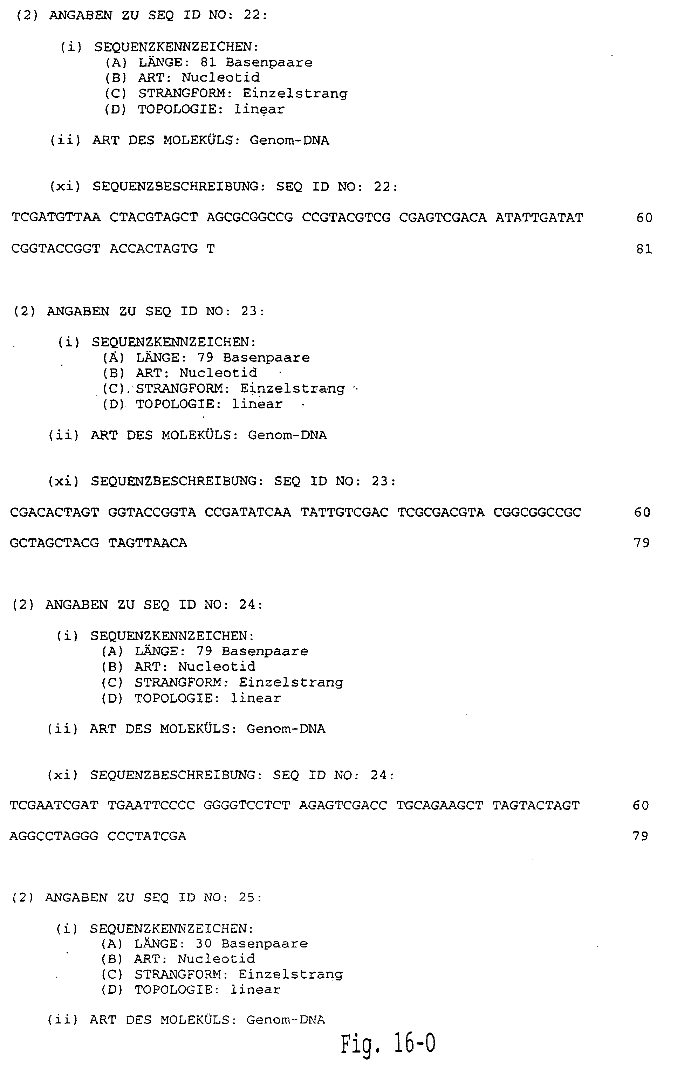

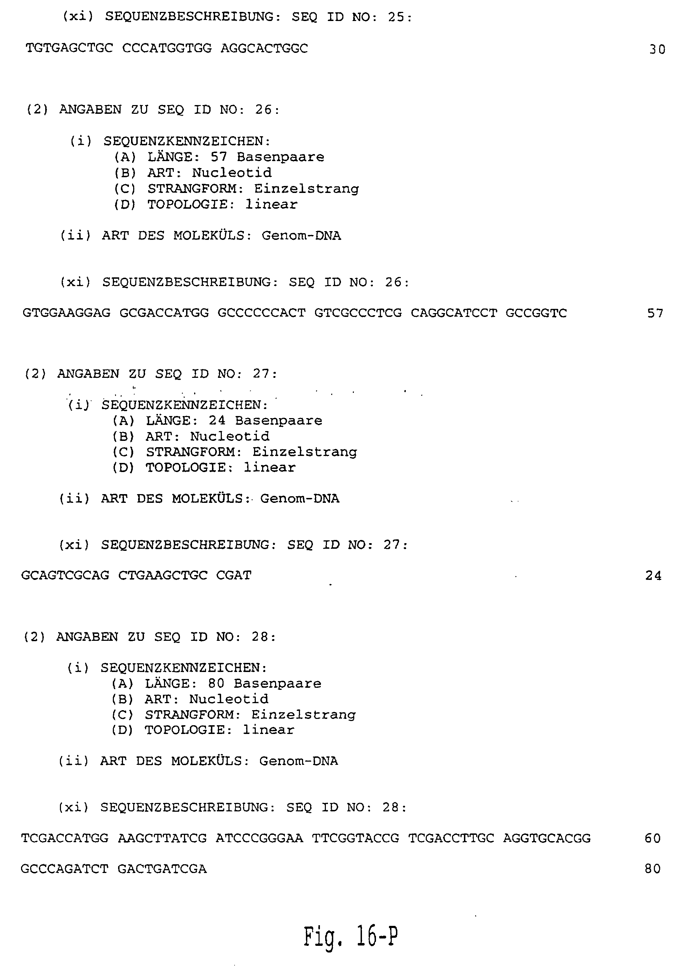

Fig. 16A-P das Sequenzprotokoll, welches gleichzeitig als Teil der Beschreibung angesehen werden soll;
Fig. 17 die schematische Darstellung des Plasmids pCMVHSA/EDHPro und
Fig. 18 eine Western Blot-Analyse von HSA-exprimierenden SK-HEP-1-Zellen. Die Zahlenwerte am Rand geben das Molekulargewicht in kDa an. Spur 1: SK-HEP-1 Negativkontrolle, Spur 2: SK-HEP-1-Klon #366, Spur 3: SK-HEP-1-Klon #368, Spur 4: SK-HEP-1-Klon #369, Spuren 5-7: plasmatische HSA-Standards, Spur 8: Molekulargewichtsstandard, Spur 9: Pichia p.-Negativkontrolle, Spur 10: HSA exprimierender Pichia p.-Produktionsstamm.

### Beispiele:

In den Beispielen wird die Klonierung der Expressionsplasmide beschrieben. Am Beispiel der Expression von Prothrombin werden die Transfektion, das Selektions- und Amplifikationsprotokoll und die dazugehörigen Kontrollexperimente beschrieben. Die Verifikation der dicistronischen mRNA wird mittels Northern Blots durchgeführt, die Amplifikation der Transkriptions-/Translationseinheit wird in Southern Blots überprüft. Für die genaue Analyse der exprimierten Fremdproteine werden Western Blots herangezogen und schließlich werden die rekombinanten Proteine mittels bekannter Koagulationstests auf ihre Aktivität hin überprüft. Die Aktivitäten werden in mUnits (mU) pro 10⁶-Zellen und 24 h angegeben. Um die allgemeine Verwendbarkeit der Expressionsplasmide zu demonstrieren, wird die Expression der Fremdproteine in verschiedenen Zellinien durchgeführt.

Beispiel 1 beschreibt die Klonierung des humanen Faktors II mit den erfindungsgemäßen Expressionsplasmiden in CHO- und 293-Zellen. Die Klonierung und Expression der Faktor VIII-Deletionsmutante FVIIIdB928 und des gesamten Faktors VIII wird in 293 und SK-HEP-1-Zellen in den Beispielen 2 und 3 beschrieben. In den weiteren Beispielen 3 bis 6 wird die Expression der humanen Faktoren von Willebrand, Faktor IX, HSA und Protein C in den Zelllinien SK-HEP-1 und 293-Zellen beschrieben. Die Zellinie SK-HEP-1 wird als Beispiel für eine humane Leberzellinie verwendet, es können aber auch andere humane Leberzellinien verwendet werden.

### Beispiel 1: Klonierung des Selektions-/Amplifikationsmarkers EMCV5.'UTR/dhfr/Hygromycin-Phosphotransferase (EDH) und dessen Anwendung auf die Expression von Faktor II

### Konstruktion der Plasmide:

pCMV: Als Ausgangsplasmid wurde pCMVβ (MacGregor und Caskey, Nucleic Acids Res. 17: 2365, 1989, Firma Clontech, Palo Alto, USA) verwendet. Dieses wurde mit NotI geschnitten, um das β-Galaktosidase-Gen zu entfernen und anschließend religiert. Daraus entstand das 3,8 kb große Plasmid pCMV.

pCMV-MCS: (MCS; Multiple Klonierungsstelle) Um überflüssige Restriktionsschnittstellen zu entfernen, wurde pCMV mit SalI und HindIII geschnitten, mit dem Klenow Fragment der E. coli DNA Polymerase I (Pol. K.) aufgefüllt und religiert. Aus dieser Reaktion entstand pCMV-MCS. Dieses Plasmid beinhaltet den "Immediate Early Gene"-Promotor/Enhancer des menschlichen CMV und 80bp der 5'UTR des zugehörigen Gens. Es schließt sich 3' eine XhoI-Schnittstelle an, gefolgt von dem SV40 16S/19S-Intron und der SV40-Polyadenylierungsstelle.

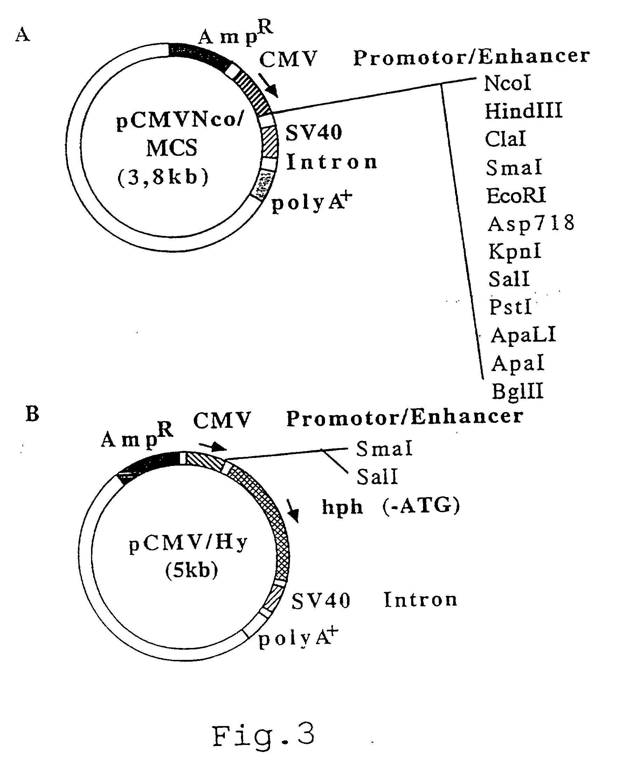

pCMVNco/MCS: pCMV-MCS wurde mit XhoI geöffnet und mit den komplementären Oligonucleotiden VI/1: 5'-TCG ACC ATG GAC AAG CTT ATC GAT CCC GGG AAT TCG GTA CCG TCG ACC TGC AGG TGC ACG GGC CCA GAT CTG ACT GAC TGA-3' (SEQ.ID.No. 9) und VI/2: 5'-TCG ATC AGT CAG TCA GAT CTG GGC CCG TGC ACC TGC AGG TCG ACG GTA CCG AAT TCC CGG GAT CGA TAA GCT TGT CCA TGG-3' (SEQ.ID.No. 10) als neue MCS ligiert. Dabei wurde die XhoI-Schnittstelle zerstört und es entstand der Vektor pCMVNco/MCS (Fig. 3-A). Die neue MCS verfügte über eine NcoI-Erkennungssequenz als Translations-Initiations-Codon um Fremdgene ohne eigenes ATG-Startcodon einsetzen und exprimieren zu können.

pCMV/Hy: In pCMVNco/MCS wurde das Hygromycin β-Phosphotransferase-(hph)-Gen ohne ATG (hph-ATG) eingesetzt. hph-ATG wurde als 1,2 kb-Fragment aus dem von Boehringer Mannheim erhältlichen Vektor pHphO als SalI, SmaI-Fragment isoliert und in die SalI- und Pol. K.-behandelte ApaLI-Schnittstellen von pCMVNco/MCS eingesetzt. Somit entstand pCMV/Hy (Fig. 3-B).

pSVDHFR: Das dhfr-Fragment samt Polyadenylierungs-Sequenz wurde als 1500bp großes PstI-Fragment aus pASDII (Kaufman and Sharp, Mol. Cell. Biol. 2: 1304, 1982) isoliert und über die PstI-Schnittstelle in pSVMCS eingesetzt. pSVMCS entstand aus pSVβ (MacGregor und Caskey, supra, Firma Clontech, Palo Alto, USA), indem das β-Galaktosidase-Gen durch Schneiden mit NotI und Religation des verbleibenden Vektors entfernt wurde. Durch Schneiden mit XbaI und HindIII, Auffüllen mit Pol.K und Religation wurde die MCS 3' der SV40-Polyadenylierungssequenz entfernt. In die NotI-Schnittstelle wurde dann eine neue MCS eingesetzt. Die eingesetzte MCS hatte folgende Sequenz: 5'-GG CCT AGG GCC'CTA GGC CTA CTA GTA CTA AGC TTC TGC AGG TCG ACT CTA GAG GAC CCC GGG GAA TTC AAT CGA TGG CC-3' (SEQ.ID.No. 11).

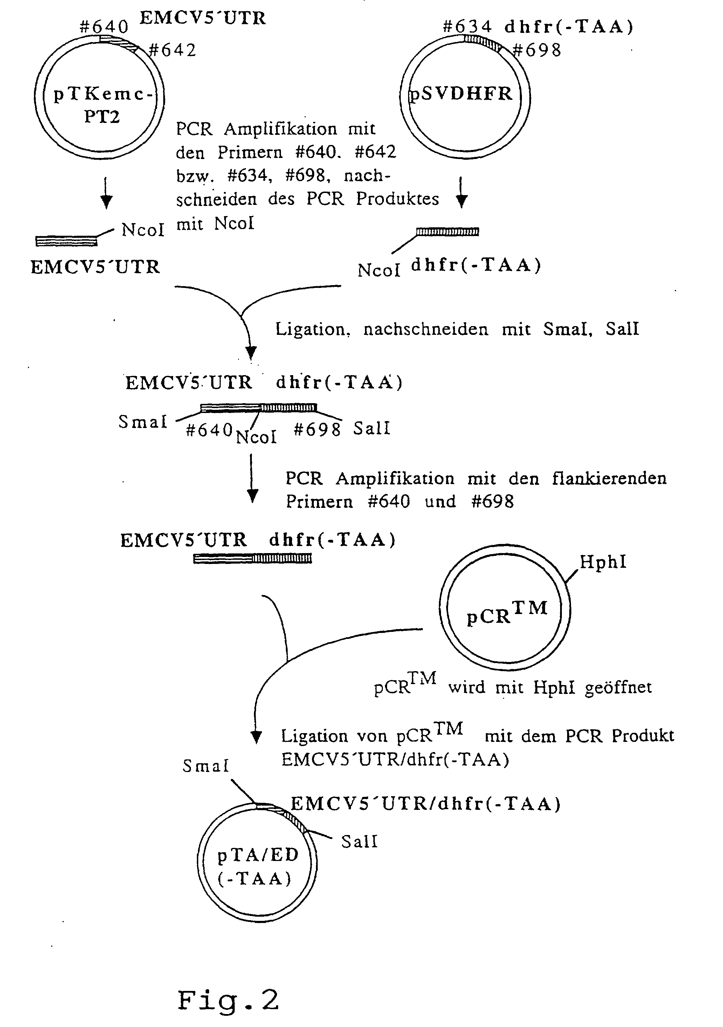

pTA/ED(-TAA) (Fig. 2): In den Vektor pCR^{TM} (Firma Invitrogen, San Diego, USA) wurde die Kassette bestehend aus dem 5' nichttranslatierten Bereich des Encephalomyocarditis Virus (EMCV5'UTR) und dem dhfr-Fragment ohne Stop-Codon TAA (-TAA) subkloniert. Die Herstellung des EMCV5'UTR/dhfr(-TAA)-Fragments erfolgte mittels Polymerase-Kettenreaktion (PCR). Das 500bp große EMCV5'UTR-Fragment wurde aus pTKemc-PT2 (WO 91/11519) durch PCR mit den Primern #640, 5'-ACC CCC GGG GGT ACC ATA TTG CCG TCT TTT GG-3' (SEQ.ID.No. 12) und #642, 5'-GGA ATT CCC ATG GTA TTA TCG TGT TTT TC-3' (SEQ.ID.No. 13) isoliert.

Das 560bp große dhfr-Fragment wurde aus pSVDHFR mittels PCR mit den Primern #634, 5'-GGA AGC TTG GCC ATG GTT CGA CCA TTG AAC TGC-3' (SEQ.ID.No. 14) und #698, 5'-GGT CAA GCT TTT CTT CTC GTA GAC TTC AAA CTT ATA CT-3' (SEQ.ID.No. 15) isoliert.

Die durch PCR-Amplifikation gewonnenen EMCV5'UTR- und dhfr-Fragmente wurden nach der gelelektrophoretischen Trennung aus "Low Melting Point Agarose" (LMA) isoliert. Die beiden Fragmente wurden jeweils mit NcoI nachgeschnitten und ligiert. Von dem Ligationsprodukt wurde neuerlich eine PCR-Amplifikation mit den flankierenden Primern angesetzt, also mit den Primern #640 und #698 (siehe oben). Das resultierende 1050bp große Fragment wurde in den Vektor pCR^{TM} (Firma Invitrogen, San Diego, USA) eingesetzt. Dabei entstand das Plasmid pTA/ED(-TAA).

pCMV/EDH-Sp: In den mit SmaI und SalI geöffneten Vektor pCMV/Hy wurde das SmaI-, SalI-Fragment EMCV5'UTR/dhfr(-TAA) aus pTA/ED (-TAA) eingesetzt. Dabei entstand das Konstrukt pCMV/EDH-Sp (Fig. 4 A).

pCMV/EDHGly: In die singuläre SalI-Schnittstelle zwischen dhfr- und hph-Gen wurde ein "Spacer" eingesetzt. Der Spacer bestand aus den komplementären Oligonucleotiden #1077 (5'-TCG ATT ACG TAC TGG AGG CGG GGG TGG AAA-3'; SEQ.ID.No. 16) und #1078 (5'-TCG ATT TCC ACC CCC GCC TCC AGT ACG TAA-3'; SEQ.ID.No. 17), verfügte über eine neue SnaBI-Schnittstelle und kodierte für fünf Glycin-Reste. Der Übergang zwischen dhfr und hph hatte somit die Sequenz: 5'-GT CGA TTA CGT ACT GGA GGC GGG GGT GGA AAT CGA CGG ATC CC-3' (SEQ.ID.No. 18).

pCMV/EDHPro: In die singuläre SalI-Schnittstelle zwischen dhfr- und hph-Gen wurde der "Spacer" von pCMV/EDHGly in reverser Orientierung eingesetzt. Somit kodierte er hier für fünf Prolin-Reste, wobei der Übergang zwischen dhfr und hph die folgende Sequenz hatte: 5'-GT CGA TTT CCA CCC CCG CCT CCA GTA CGT AAT CGA CGG ATC CC-3' (SEQ.ID.No. 19).

pCMVFII/EDH-Sp (Fig. 4 B): Die Faktor II-cDNA wurde als 2 kb-Fragment aus pTKemc-PT2 (WO 91/11519) isoliert, indem mit NcoI partial und SmaI vollständig geschnitten wurde. Dieses Fragment wurde in den Vektor pCMV/EDH-Sp eingesetzt, welcher ebenfalls NcoI partial und SmaI vollständig geschnitten wurde.

pCMVFII/EDHGly: Die Faktor II-cDNA wurde als 2 kb-Fragment aus pTKemc-PT2 (WO 91/11519) isoliert, indem mit NcoI partial und SmaI vollständig geschnitten wurde. Dieses Fragment wurde in den Vektor pCMV/EDHGly eingesetzt, welcher ebenfalls NcoI partial und SmaI vollständig geschnitten wurde.

pCMVFII/EDHPro: Die Faktor II-cDNA wurde als 2 kb-Fragment aus pTKemc-PT2 (WO 91/11519) isoliert, indem mit NcoI partial und SmaI vollständig geschnitten wurde. Dieses Fragment wurde in den Vektor pCMV/EDHPro eingesetzt, welcher ebenfalls NcoI partial und SmaI vollständig geschnitten wurde.

### Herstellung der permanenten Zellinien:

Initialselektion: CHO-(ATCC CRL 9096) und 293-Zellen (ATCC CRL 1573) wurden von der American Type Culture Collection (Rockville, MD) bezogen. Beide Zellinien wurden mit den Konstrukten pCMVFII/EDH-Sp, pCMVFII/EDHGly und pCMVFII/EDHPro entsprechend Graham and von der Eb, Virology 52: 456, 1973 transfiziert. CHO-Zellen wurden der DHFR-Selektion, der Hygromycin B-Selektion und der gleichzeitigen Hygromycin B- (HyB) und DHFR-Selektion unterzogen. 293-Zellen wurden der Hygromycin B-Selektion ausgesetzt. Nach 10-20 Tagen wurden die resistenten Kolonien vereinzelt und auf Faktor II (FII)-Expression getestet.

DHFR-Selektionsmedium: DMEM/HAMs F12 ohne Glycin Thymidin und Hypoxanthin, 10% dialysiertes fötales Kälberserum, 10IU/ml Penicillin, 100µg/ml Streptomycin (Gibco 043-05140H), L-Glutamin (Gibco 043-05030H).

Hygromycin B-Selektionsmedium: DMEM/HAMs F12, 10% fötales Kälberserum, 10IU/ml Penicillin, 100µg/ml Streptomycin (Gibco 043-05140H), L-Glutamin (Gibco 043-05030H), je 10µg/ml Adenosin, Thymidin und Deoxyadenosin (Sigma), 200µg Hygromycin B (Calbiochem)/ml.

Genamplifikation: Die Amplifikation via hph erfolgte mittels Hygromycin B (HyB) beginnend bei 200µg HyB/ml. Um die Möglichkeit von Rearrangements oder Deletionen durch zu hohe Konzentrationen von HyB gering zu halten, wurde die HyB-Konzentration je Amplifikationsschritt jeweils nur verdoppelt. Die Amplifikation mittels DHFR bei CHO-Zellen erfolgte beginnend bei 10nM Methotrexat (MTX) durch Verdopplung der MTX-Konzentration je Stufe. Die Amplifikation von 293 Zellen wurde beginnend bei 100nM MTX angesetzt. Die bei jeder Amplifikationsstufe entstehenden resistenten Zellklone wurden vereinzelt und auf Faktor II-Expression untersucht.

Bestimmung der Faktor II-Aktivität: Die zu testenden Faktor IIexprimierenden Zellklone wurden mit serumfreiem Testmedium, supplementiert mit 5µg/ml Vitamin Kl 24 Stunden inkubiert. Die Bestimmung der Gerinnungsaktivität erfolgte mit einem Koagulometer KC4A (Firma Amelung GmbH, BRD) nach einer modifizierten Prothrombin-Zeit-Methode (Falkner et al. 1992).

Proteinnachweis mittels Western Blot-Analysen: Western Blots wurden entsprechend Towbin et al., PNAS 76: 4350, 1979 vorgenommen. Als erster Antikörper wurde ein Anti-Prothrombin-Antikörper (Firma Dakopatts, Dänemark) in einer Verdünnung von 1:100 eingesetzt. Als zweiter Antikörper wurde ein Ziegen-Anti-Kaninchen-Antikörper (Firma BioRad, CA, USA) in einer Verdünnung von 1:7500 verwendet, welcher mit alkalischer Phosphatase konjugiert war. Der Nachweis mittels Färbung erfolgte nach Standardmethoden mit dem Protoblot-System der Fa. Promega.

Untersuchungen der DNA- und RNA-Struktur: Die Präparation der zellulären DNA erfolgte nach Gross-Bellard et al., Eur. J. Biochem. 36: 32, 1973, Southern Blot-Analysen nach Southern, (J. Mol. Biol. 98: 503, 1975) bzw. nach Sambrook et al., Molecular Cloning, A Laboratory Manual, Second Edition, Cold Spring Harbor Laboratory Press 1989. Die für die Spaltung der zellulären DNA notwendigen Restriktionsenzyme wurden von Boehringer Mannheim, BRD bezogen. Die Hybridisierungssonden Faktor II, dhfr und hph wurden aus den Plasmiden pCMVFII/EDHPro, pSVDHFR und pCMV/Hy präpariert.

Die Isolierung der mRNA erfolgte mit den Materialien und nach den Protokollen der Firma Invitrogen, USA ("Fast Track", #1-800-955-6288), Northern Blot-Analysen wurden entsprechend Sambrook et al., supra, durchgeführt. RT-PCR Analysen erfolgten mit den Materialien der Firma Perkin Elmer Cetus, USA ("rTth Reverse Transcriptase RNA PCR Kit", #N808-0069) nach Kwok, PCR Protocols. A Guide to Methods and Applications. Academic Press, Inc., San Diego, CA 1990 bzw. Myers et al., Biochemistry 30: 7661, 1991, wobei je Reaktion 2µg mRNA eingesetzt wurden. Als Primer wurden #1489 (bindet 3' in der Faktor II-cDNA): 5' GGA AAT ATG GCT TCT ACA CAC ATG TGT TCC GCC TGA A 3' (SEQ.ID.No. 20) und #1490 (bindet 5' im dhfr- Gen): 5' TCC GTT CTT GCC AAT CCC CAT ATT TTG GGA CAC GGC G 3' (SEQ.ID.No. 21) eingesetzt.

Konstruktion des Selektions-/Amplifikationsmarkers EMCV5' UTR/dhfr/Hygromycin Phosphotransferase (EDH): Das CHO-Zellexpressionssystem ist in seiner üblichsten Ausführung mit DHFR-Selektion bzw. Methotrexat (MTX)-Amplifikation verbunden und an die Verfügbarkeit von DHFR-defizienten Zellinien wie CHO DUKX B11 (Urlaub and Chasin, supra) gebunden. Da sich CHO-Zellen jedoch nicht bedingungslos für die Expression jedes beliebigen Proteins eignen, wurde der Versuch unternommen, auch andere Zellinien als Expressionssysteme effizient nutzbar zu machen. In diesem Sinne wurde der EDH-Marker konstruiert. Er findet seine Hauptanwendung in solchen Zellen, die über ein endogen funktionelles dhfr-Gen verfügen, da in derartigen Zellinien die Selektion bzw. Genamplifikation durch DHFR bzw. MTX nur unzureichend durchführbar ist.

Bei diesem EDH-Marker handelte es sich um ein bifunktionelles Fusionsprotein, welches sich aus dem Dihydrofolat Reduktase (dhfr)-Gen und dem Hygromycin Phosphotransferase (hph)-Gen zusammensetzt. Das hph-Gen wurde gewählt, weil es einen sehr guten Selektionsmarker darstellt, das dhfr-Gen deshalb, weil es den besten Amplifikationsmarker darstellt.

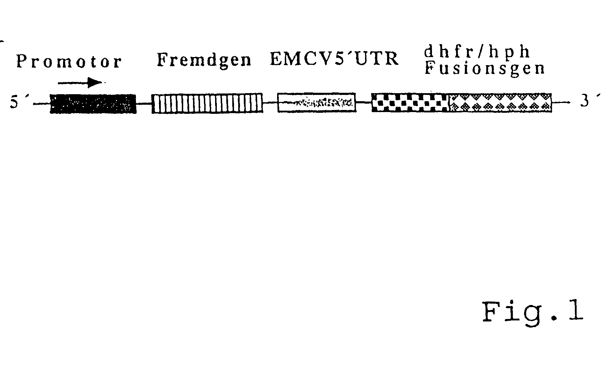

Da nicht auszuschließen war, daß sich die beiden fusionierten enzymatischen Proteineinheiten durch ihre räumliche Nähe in ihrer Aktivität beeinflussen oder sogar behindern könnten, wurde versucht, dies dadurch zu verhindern, daß zwischen die beiden Fusionsprotein-Anteile ein sogenannter "Spacer" eingesetzt wurde. Bei diesem Spacer handelte es sich um ein kurzes Oligonucleotid, welches in der einen Orientierung eingesetzt für fünf Glycin-Reste ("Glycin-Spacer", Gly) in der reversen Orientierung für fünf Prolin-Reste ("Prolin-Spacer", Pro) codierte. Mit der gewählten Anordnung von dem zu exprimierenden Fremdgen und Fusionsmarkergen sollte eine dicistronische RNA gebildet werden können. Dies wurde erreicht, indem an das 5'-Ende des Fusionsmarkers auf DNA-Ebene eine Sequenz eingesetzt wurde, die als "internal ribosome entry site" (IRES) fungierte. Hier kam die IRES des Encephalomyocarditis-Virus (EMCV) zur Anwendung. Sie befindet sich im 5'-nicht-translatierten Bereich (5'UTR) des EMCV und wird deshalb EMCV5'UTR genannt. Die resultierende Gen-Kassette, bestehend aus EMCV5'UTR/-dhfr/hph (EDH), wurde 3' des zu exprimierenden Fremdgens angeordnet, sodaß sich die in Fig. 1 dargestellte Konfiguration von Promotor, Fremdgen und EDH-Kassette ergab.

Für das Fusionsprotein des EDH-Selektions-/Amplifikationsmarkers wurde die EMCV5'UTR/dhfr (ED)-Kassette via PCR kloniert. Das EMCV5'UTR-Fragment wurde aus dem Plasmid pTKemc-PT2 (WO 91/11519), das dhfr-Fragment (ohne Stop Codon TAA) aus dem Plasmid pSVDHFR mittels PCR isoliert, die beiden Amplifikationsprodukte mit NcoI nachgeschnitten, ligiert und das Ligationsprodukt neuerlich mittels PCR amplifiziert und in der Folge in den Vektor pCR^{TM} (Firma Invitrogen, San Diego, USA) subkloniert. Das Konstruktionsschema ist in Fig. 2 dargestellt. Aus dem resultierenden Plasmid pTA/ED (-TAA) wurde die Kassette EMCV5'UTR/dhfr (-TAA) isoliert und in das Plasmid pCMV/Hy (Fig. 3-B) eingesetzt. Das Plasmid pCMV/Hy verfügte bereits über das Hygromycin Phosphotransferase-Gen (aus pHphO, Boehringer Mannheim, BRD) ohne Start-Codon (hph-ATG). Durch diese Vorgangsweise entstand die 2,2 kb umfassende Genkassette EDH in Form des Konstruktes pCMV/EDH-Sp (Fig. 4-A). In diesem Plasmid lag das dhfr-Gen in unmittelbarer Fusion mit dem hph-Gen vor. Um eine potentielle Behinderung der beiden Komponenten DHFR und Hygromycin Phosphotransferase (HPH) auf Proteinebene zu verhindern, wurde zwischen die beiden Gene ein kurzes Oligonucleotid als "Spacer" eingesetzt. Somit entstanden die drei Varianten des Selektions-/Amplifikationsmarkers EDH-Sp, EDHGly und EDHPro. In Fig. 4-A ist repräsentativ das Expressionsplasmid pCMV/EDH-Sp dargestellt, die beiden anderen Expressionsplasmide wurden als pCMV/EDHGly bzw. pCMV/EDHPro bezeichnet.

In diese drei Ausgangsvektoren wurde als "gene of interest" die Faktor II-cDNA als 2 kb umfassendes NcoI-SmaI-Fragment aus pTKemc-PT2 (WO 91/11519) eingefügt, wodurch die Expressionsplasmide pCMVFII/EDH-Sp, pCMVFII/EDHGly und pCMVFII/EDHPro entstanden. Repräsentativ ist pCMVFII/EDH-Sp in Fig. 4-B gezeigt.

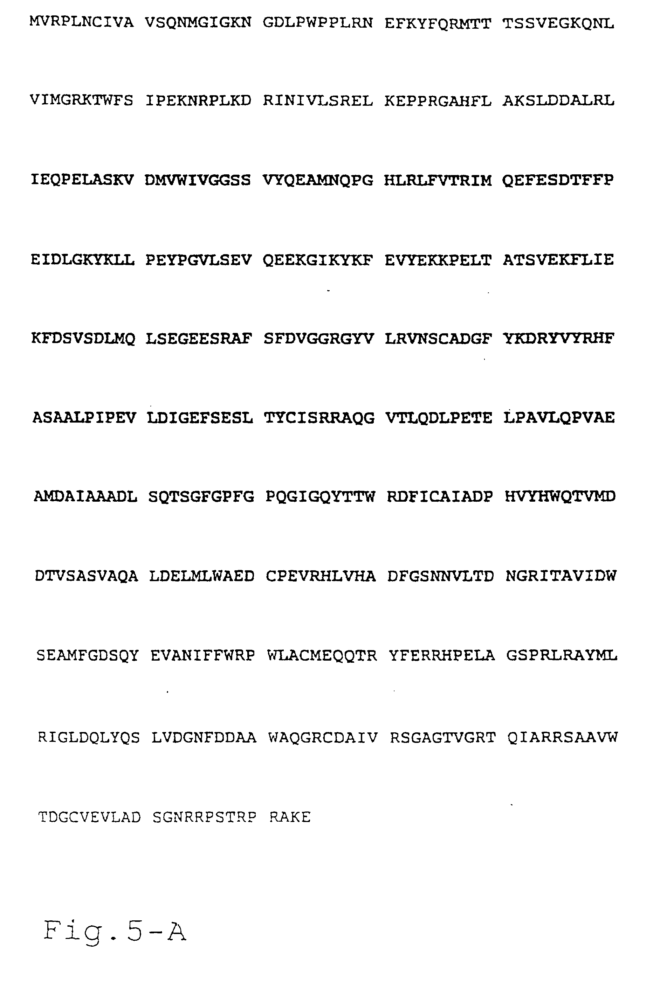

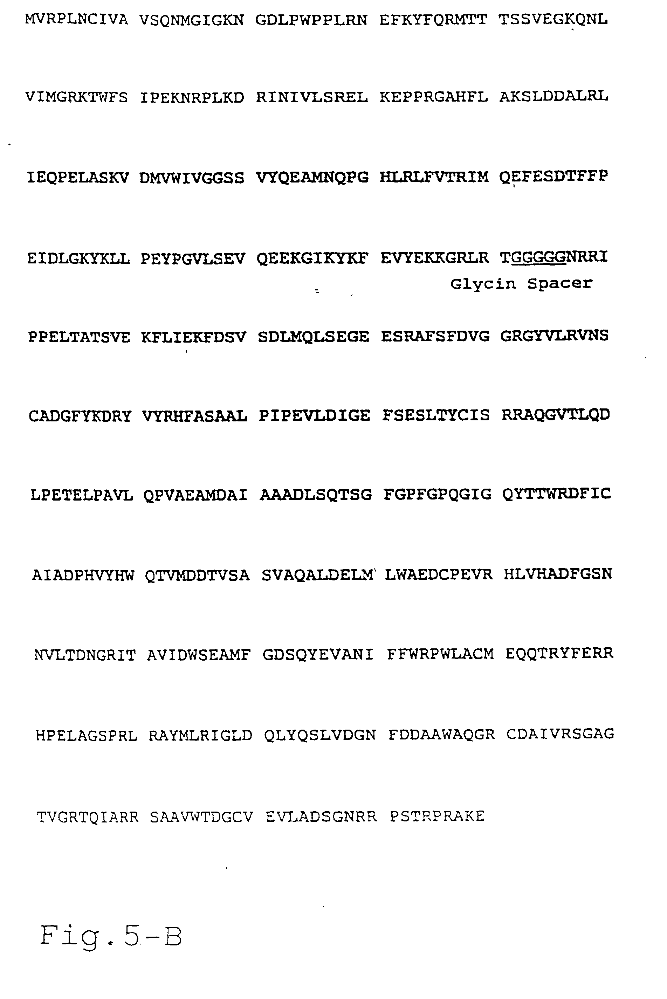

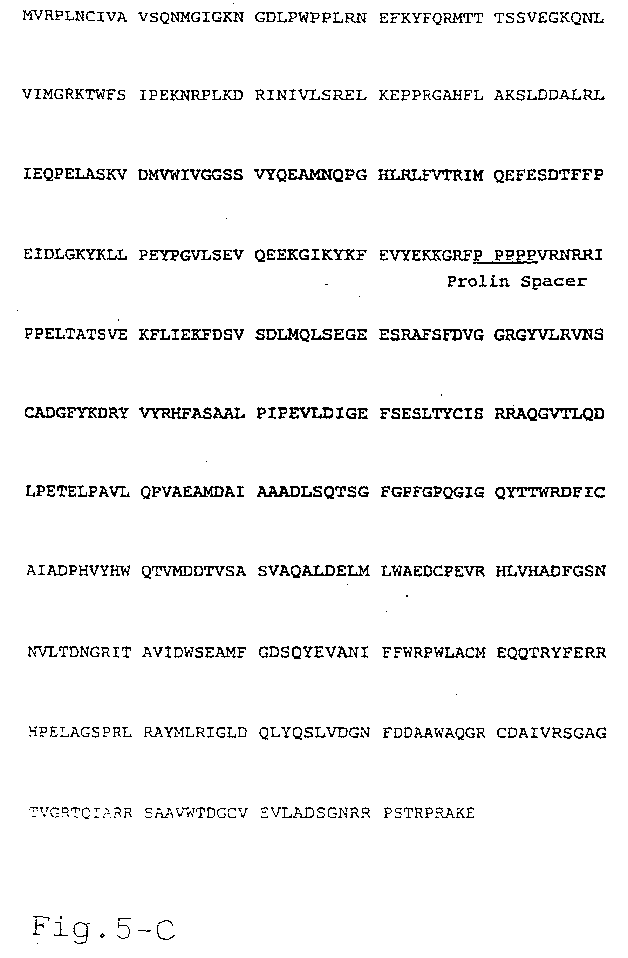

Die Aminosäuresequenzen der Fusionsproteine DHFR/HPH-Sp, DHFR/HPHGly und DHFR/HPHPro sind in Fig. 5-A, B und C dargestellt.

Untersuchung der funktionellen Eigenschaften des EDH-Selektions-/Amplifikationsmarkers in transfizierten Zellen: Die drei Konstrukte pCMVFII/EDH-Sp, pCMVFII/EDHGly und pCMVFII/EDHPro wurden hinsichtlich ihrer Eigenschaften zur Selektion und Amplifikation untersucht. Zu diesem Zweck wurden sie in CHO- und 293-Zellen transfiziert. In DHFR-defizienten CHO-Zellen (Urlaub and Chasin, 1980) wurde das getrennte und das gleichzeitige Funktionieren der beiden Fusionsprotein-Komponenten DHFR und HPH getestet. Die transfizierten 293-Zellen, als Repräsentanten einer DHFR positiven Zellinie wurden hinsichtlich der Funktion der HPH-Komponente untersucht, indem sie mit dem Antibiotikum Hygromycin B (HyB) selektiert wurden. Die Ergebnisse der DHFR- und HPH-Initialselektion sind in Tabelle 1 zusammengefaßt.

**Tabelle 1**

| | CHO-Zellen/ EDH-System mUnits (µg)/ 10⁶ -Zellen | CHO-Cotransfektion (Jorgensen et al., 1987) mUnits (µg)/ 10⁶ -Zellen | 293-Zellen mUnits (µg)/ 10⁶-Zellen |
|---|---|---|---|
| Initialselektion | 12-15 (1,2-1,5) | (0,1) | 50-55 (5-5,5) |
| Amplifikation | / | / | / |
| 100nM MTX | / | / | 100-150 (10-15) |
| 150nM MTX | 150-160 (15-16) | / | / |
| 1000nM MTX | / | 8-11 (1,3-1,6) | / |

Sie zeigen, daß CHO-Zellen initial zwischen 12-15mU Faktor II/10⁶- Zellen und 24 Stunden exprimieren, mit 293-Zellen ließen sich Werte bis zu 55mU Faktor II/10⁶-Zellen und 24 Stunden nachweisen. Das erfindungsgemäße Expressionssystem zeigt also in CHO-Zellen nach initialer Selektion eine unerwartet hohe Expression an Faktor II. Diese hohe Expression von Faktor II konnte allerdings bei Verwendung der Zellinie 293 noch weiter gesteigert werden.

Die aus der Initialselektion resultierenden Zellklone wurden auf das Amplifikationsvermögen der DHFR- und HPH-Komponente des EDH-Markers untersucht. Die Ergebnisse daraus sind ebenfalls in Tabelle 1 zusammengefaßt. Auch hier ließ sich zeigen, daß bereits bei 100nM MTX mit 293 Zellen gleichviel Faktor II exprimiert wird verglichen mit CHO-Zellen, die auf 150nM MTX wuchsen.

Die Bildung dicistronischer RNA und das Funktionieren des EDH-Markers wurde anhand der Expression von Faktor II untersucht. Die initial selektierten transfizierten CHO- und 293-Zellklone zeigten in der Northern Blot-Analyse und in der RT (Reverse Transkriptase)-PCR das Vorhandensein dicistronischer RNAs.

Sowohl die initial selektierten als auch die amplifizierten CHO- und 293-Zellklone wurden mittels Southern Blot-Analyse auf ihre genomische Struktur untersucht. Die initialselektierten Zellklone zeigten eine Kopienanzahl, die im Bereich von 1-5 Genkopien/Zelle lag. Im Rahmen der Amplifikation über die HPH Komponente des EDH-Markers konnte ausgehend von 200µg HyB/ml bis auf 3000µg HyB/ml eine leichte Genamplifikation festgestellt werden (siehe auch Beispiel 2).

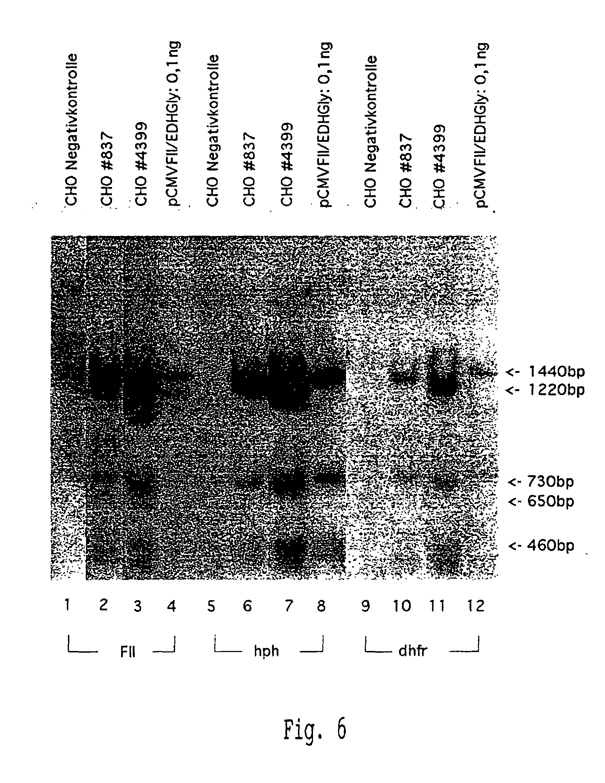

Die Amplifikation über die DHFR-Komponente des EDH-Markers wurde untersucht, indem transfizierte CHO-Zellen ausgehend von 10nM MTX einer sukzessive steigenden MTX-Konzentration bis 40nM ausgesetzt wurden. Trotz dieser sehr geringen Anhebung der MTX-Konzentration konnte bereits klar Genamplifikation nachgewiesen werden (Fig. 6). Dies wird deutlich, wenn die Signalintensitäten des DHFR initial selektierten CHO-Klones #837 mit denen des daraus hervorgegangenen, auf 40nM MTX-amplifizierten CHO-Klones #4399 verglichen werden. Dieser Effekt läßt sich sowohl bei Hybridisierung mit einer Faktor II-spezifischen Sonde (#837 in Spur 2 und #4399 in Spur 3) als auch mit einer hph (#837 in Spur 6 und #4399 in Spur 7) und dhfr (#837 in Spur 10 und #4399 in Spur 11) spezifischen Sonde nachweisen. Die Spuren 1, 5 und 9 stellen jeweils die Negativkontrollen aus nicht transfizierten CHO-Zellen dar. In den Spuren 4, 8 und 12 wurde das Referenzplasmid pCMVFII/EDHGly aufgetragen.

Der Effekt der Genamplifikation über die DHFR-Komponente des EDH-Markers konnte in gleicher Weise sowohl in initial DHFR-selektierten CHO-Zellen (Fig. 6), als auch in initial HyB-selektierten Zellen festgestellt werden.

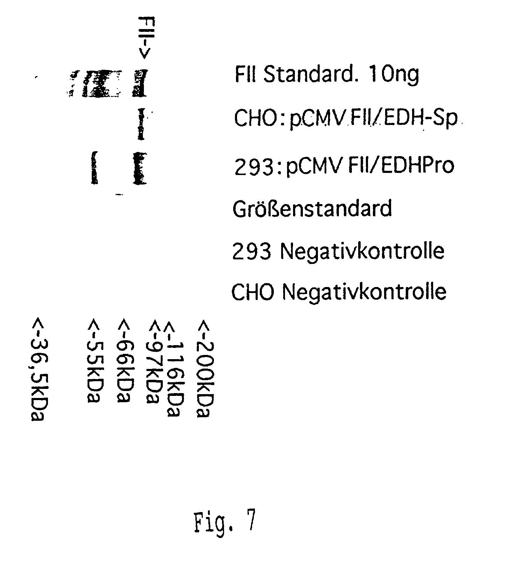

Expression von Faktor II: Die Identität des exprimierten Faktor II mit seinem plasmatischen Analogon wurde im Rahmen von Western Blot-Analysen bestätigt (Fig. 7). Die Zahlenwerte am Rand geben die Molekulargewichte in kDa an. Die Faktor II-spezifische Bande ist mit einem Pfeil markiert.

Mit den vorgenommenen Amplifikationen ging auch eine Erhöhung der Faktor II-Expression einher. Anfangs betrug die Expression von Faktor II in CHO-Zellen 12-15mU/10⁶-Zellen und 24 h (entspricht mindestens 1,2-1,5µg Faktor II/10⁶-Zellen und 24 h). Mit dem hier beschriebenen System konnte also eine Steigerung von zumindest einem Faktor von 10 gegenüber der Literatur erreicht werden. Mit 293-Zellen wurden Initial-Werte von 50-55mU (entspricht mindestens 5-5,5µg Faktor II)/10⁶-Zellen und 24 h erzielt, wobei 293-Zellen wiederholt in dieser Größenordnung signifikant mehr Faktor II exprimierten als CHO-Zellen.

Die Amplifikation in CHO-Zellen ergab bei 150nM Methotrexat (MTX) Expressionen im Bereich von 150-160mU (entspricht mindestens 15-16 µg Faktor II)/10⁶-Zellen und 24 h. Trotz dieses relativ geringen Amplifikationsniveaus konnten also im Vergleich mit der Literatur deutlich höhere Werte erreicht werden. Bei den hier beschriebenen Angaben von mindestens 15-16µg Faktor II bei 150nM MTX handelte es sich bereits um Aktivitätswerte, sodaß die Expressionssteigerung mit dem hier beschriebenen System beträchtlich war. Bei der von Jorgensen beschriebenen Methodik wurde trotz Amplifikation in einer sieben Mal höheren MTX-Konzentration nur ein Zehntel der erfindungsgemäßen Expressionswerte erreicht. Zusätzlich ist zu berücksichtigen, daß ausgehend von 150nM MTX noch ein großes MTX-Amplifikationspotential offen ist.

Die Expressionswerte, die in CHO- und 293-Zellen mit dem hier beschriebenen EDH-Marker Expressionssystem und dem konventionellen System der CHO-Cotransfektion (Jorgensen et al., supra) erreichbar waren, sind vergleichend in Tabelle 1 dargestellt.

### Beispiel 2: Expression von komplettem Faktor VIII (FVIIIc) und der Deletionsmutante FVIIIdB928 in transformierten in 293- und SK-HEP-1-Zellen

### Konstruktion der Plasmide:

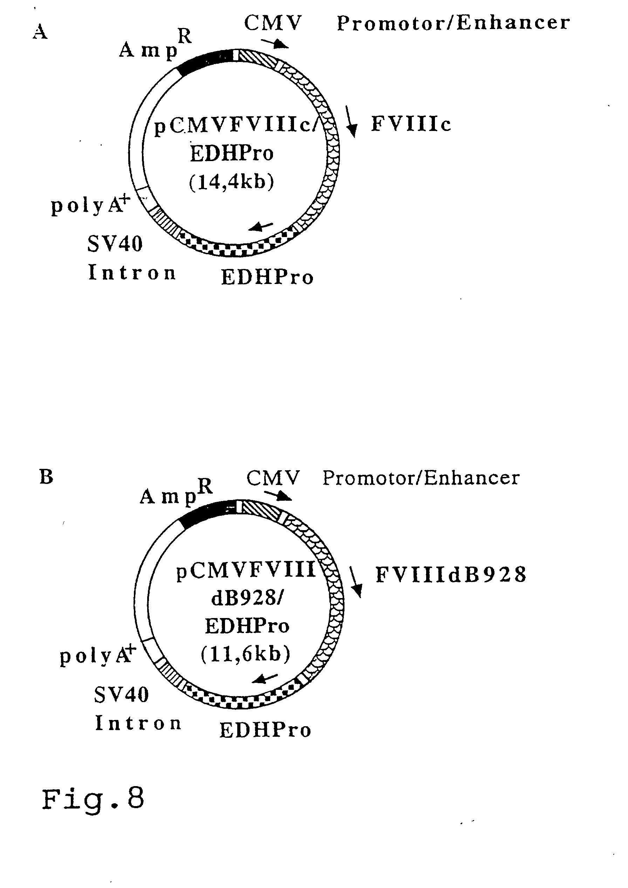

pCMVFVIIIc/EDHPro (Fig. 8-A): Die volle Länge Faktor VIII-cDNA wurde von Leyte et al., Biochem. J. 263: 187, 1989 konstruiert. Die 7,2 kb umfassende Faktor VIII-cDNA wurde als Fragment mit glatten Enden in die SmaI-Schnittstelle von pCMV/EDHPro (siehe Beispiel 1) eingesetzt. Daraus resultierte das Expressionsplasmid pCMVFVIIIc/EDHPro.

pCMVFVIIIdB928/EDHPro (Fig. 8-B): Die Deletion der Faktor VIII B-Domäne ist bei Leyte et al., J. Biol. Chem. 266: 740, 1991 beschrieben. Die 4,4 kb umfassende FVIIIdB928-cDNA wurde als Fragment mit glatten Enden in die SmaI-Schnittstelle von pCMV/EDHPro (siehe Beispiel 1) eingesetzt.

Herstellung der permanenten Zellinien: Initialselektion: 293-Zellen (ATCC CRL 1573) wurden von der American Type Culture Collection (Rockville, MD, USA) bezogen, mit den Konstrukten pCMVFVIIIdB928/EDHPro bzw. pCMVFVIIIc/EDHPro nach Graham and van der,Eb, supra transfiziert und der HyB-Selektion unterzogen (siehe Beispiel 1). Nach 10-20 Tagen wurden die resistenten Kolonien vereinzelt und auf Faktor VIII-Expression getestet.

SK-HEP-1-Zellen (ATCC HTB 52) wurden von der American Type Culture Collection (Rockville, MD, USA) bezogen und mit den Konstrukten pCMVFVIIIdB928/ EDHPro bzw. pCMVFVIIIc/EDHPro transfiziert. Die Transfektion erfolgte nach Neumann et al., EMBO J. 1:841, 1982 in modifizierter Form. Dabei wurden 1-3x10⁷-Zellen für einen Elektroporationsansatz eingesetzt, wobei der Puls mittels eines BioRad Gene Pulsers^{TM} (Firma BioRad, CA, USA) bei 1000V, 25µF, 200 Ohm durchgeführt wurde. Anschließend an den Puls wurden die Zellen in Medium aufgenommen und 48 Stunden nach dem Puls in HyB-Selektionsmedium (siehe Beispiel 1) überführt. Nach 10-20 Tagen wurden die resistenten Kolonien vereinzelt und auf Faktor VIII-Expression getestet.

Genamplifikation: Die Amplifikation mittels Hygromycin B (HyB) erfolgte beginnend bei 200µg HyB/ml durch Verdopplung der HyB-Konzentration je Stufe (siehe Beispiel 1). Die Amplifikation mittels DHFR bei 293 und SK-HEP-1-Zellen erfolgte beginnend bei 100nM Methotrexat (MTX) durch Verdopplung der MTX-Konzentration je Stufe. Die bei jeder Amplifikationsstufe entstehenden resistenten Zellklone wurden vereinzelt und auf Faktor VIII-Expression untersucht.

Aktivitätsbestimmung von Faktor VIII: Sämtliche Aktivitätstests erfolgten mit den Materialien ("COATTEST VIII:C/4") und nach dem Protokoll der Firma Chromogenix AB, Schweden bzw. mit dem "Immunochrom FVIII:C" Kit der Firma Immuno, Österreich.

Proteinnachweis mittels Western Blot-Analysen: Western Blots wurden entsprechend Towbin et al., (supra) vorgenommen. Als erster Antikörper wurde eine Mischung der monoklonalen Anti-Faktor VIII-Antikörper CLB Cag A, CLB Cag 9 und CLB Cag 117 (alle drei Stel et al., Blood 63: 1408, 1983) verwendet. Als zweiter Antikörper wurde ein Ziege-Anti-Maus-Antikörper (Firma BioRad, CA, USA) in einer Verdünnung von 1:7500 verwendet, welcher mit alkalischer Phosphatase konjugiert war. Der Nachweis mittels Färbung erfolgte nach Standardmethoden mit dem Protoblot-System der Firma Promega.

Untersuchungen der DNA und RNA Struktur: Die Präparationen von DNA und RNA erfolgten wie bei Beispiel 1 beschrieben. Zur Hybridisierung im Rahmen der Southern Blot-Analysen bzw. Northern Blot-Analysen wurden Faktor VIII-, dhfr- und hph-Fragmente aus den jeweiligen Plasmiden isoliert (also aus pCMVFVIIIc/EDHPro, pSVDHFR und pCMV/Hy).

Für die Expression von Faktor VIII wurden bisher besonders CHO-Zellen untersucht (Kaufman et al., J. Biol. Chem. 263: 6352, 1988; Pittman et al., Blood 81: 2925, 1993). Die DHFR-defizienten CHO-Zellen waren zwar insofern interessant, als sie sich leicht über DHFR selektieren bzw. mit MTX hoch amplifizieren lassen. Der entscheidende Nachteil im Rahmen der Nutzung von CHO-Zellen bestand jedoch darin, daß sie nur außerordentlich geringe Mengen an Faktor VIII exprimierten, vor allem nach initialer Selektion konnte kein Faktor VIII nachgewiesen werden. Die Isolierung von Faktor VIII exprimierenden CHO-Zellinien erfordert also hohe Amplifikation. Dies ist mit sehr hohem "Screening"-Aufwand verbunden, da die Amplifikation "blind", das heißt ohne vorherigem Testen von initial selektierten Zellklonen ablaufen muß. Zudem erwies es sich als schwierig, stabil Fremdprotein-exprimierende CHO-Zellinien zu etablieren, da es häufig zum Auftreten von "double minute" Chromosomen kommt (Schimke et al., Cold Spring Harbor Symp. Quant. Biol. 45, 1981; Kaufman et al., Mol. Cell. Biol. 3: 699, 1983). Auch aus diesem Grund kann stabile Fremdprotein-Expression mit dem CHO-Zellsystem nur durch häufiges und aufwendiges Subklonieren der untersuchten Zellklone erreicht werden, was jedoch umso aufwendiger wird, je höher die jeweiligen Zellklone amplifiziert werden.

Aus diesen Gründen sollten außer CHO-Zellen, andere, DHFR-positive Zellinien auf ihr Faktor VIII-Expressionsvermögen untersucht werden. Präferenziell sollten dabei humane Zellinien verwendet werden, um mögliche Spezies-abhängige Änderungen der notwendigen posttranslationalen Modifikationen auszuschließen. Um diese DHFR-positiven Zellinien einerseits effizient selektieren zu können und andererseits via dhfr amplifizieren zu können, wurde der EDHPro-Selektions-/Amplifikationsmarker eingesetzt. Als Zellinien wurden vergleichend 293 und SK-HEP-1-Zellen herangezogen. Nachdem Faktor VIII endogen zum Teil in der Leber synthetisiert wird, wurden stellvertretend für humane Leberzellen SK-HEP-1-Zellen verwendet.

Über die Zellinie SK-HEP-1 als Expressionsvehikel gibt es bisher in der Literatur keine Hinweise. Die Zellinie 293 wurde bereits für die Expression von Protein C verwendet (Walls et al., 1989) und hatte sich für die Expression von Faktor II bewährt (siehe Beispiel 1). Beide Zellinien wurden jedoch noch nicht für die Expression von Faktor VIII untersucht bzw. beschrieben.

Obwohl zwar auch die komplette Faktor VIII-cDNA (FVIIIc) herangezogen wurde, lag der Schwerpunkt auf der Expression einer Faktor VIII-Mutante (FVIIIdB928), welche die gesamte B-Domäne deletiert hatte (Leyte et al., J. Biol. Chem. 266: 740, 1991).

Die Konstruktion des EDH-Selektions-/Amplifikationsmarkers erfolgte wie in Beispiel 1 beschrieben. Das Expressionsplasmid pCMVFVIIIc/EDHPro (Fig. 8-A) entstand, indem die vollständige Faktor VIII-cDNA als Fragment mit glatten Enden in pCMV/EDHPro eingesetzt wurde.

Analog entstand pCMVFVIIIdB928/EDHPro.

Herstellung und Analyse der pCMVFVIII/EDHPro-transfizierten Zellinien: Die Zellinien 293 bzw. SK-HEP-1 wurden mit den Konstrukten pCMVFVIIIdB928/EDHPro bzw. pCMVFVIIIdB928/EDHPro und pCMVFVIIIc/EDHPro transfiziert und der HyB-Selektion unterzogen. Die daraus resultierenden Zellklone wurden entsprechend Beispiel 1 auf ihre RNA-Struktur untersucht. Die gebildeten RNAs lagen dicistronisch vor. Die Abschätzung der vorhandenen Gen-Kopienanzahl wurde mittels Southern Blot-Analyse durchgeführt und bewegte sich bei den untersuchten 293 Zellen im Bereich von 1-2, bei den untersuchten SK-HEP-1-Zellen bei 5-10.

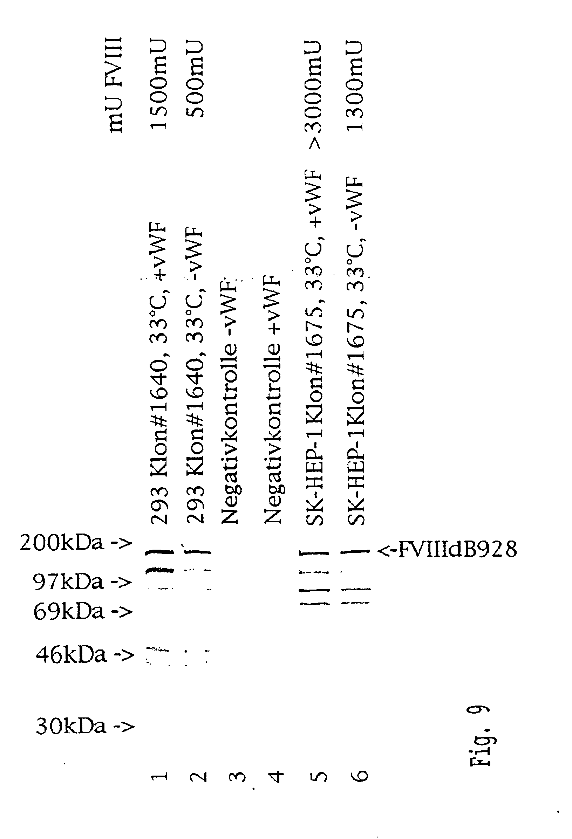

Die Amplifikation der transfizierten FVIIIdB928-exprimierenden SK-HEP-1 Zellen via hph von 200µg HyB/ml auf 1500µg HyB/ml zeigte deutlich den Effekt der Genamplifikation, wie es in Fig. 9 dargestellt ist. Die TaqI-geschnittene zelluläre DNA des initial selektierten Zellklones #1963 wurde der des daraus hervorgegangenen, auf 1500µg HyB/ml amplifizierten Zellklones #3310 gegenübergestellt. Nach allen Hybridisierungen mit einer Faktor VIII (Spur 1-4), dhfr (Spur 9-12) und hph (Spur 5-8) spezifischen Sonde zeigte sich dabei eine Verstärkung der Signalintensitäten bei Klon #3310 im Vergleich zu #1963. Der interne Standard ist durch die Reaktion der endogenen Faktor VIII-Banden gegeben. Durch den Vergleich dieser endogenen Faktor VIII-Banden der SK-HEP-1 Negativkontrolle (Spur 1) mit denen der Klone #1963 (Spur 2) und #3310 (Spur 3) ist auch die Abschätzung der vorhandenen Faktor VIII Gen-Kopien bzw. die Angleichung der aufgetragenen DNA-Menge möglich. In den Spuren 4, 8 und 12 ist jeweils das Referenzplasmid pCMVFVIIIdB928/EDHPro dargestellt.

Für die Umstellung von HyB-Selektion auf DHFR-Amplifikation wurde als optimale MTX-Konzentration 100nM MTX ermittelt. Die anschließende Amplifikation erfolgte entsprechend dem Prinzip der üblichen DHFR-Amplifikation (siehe Beispiel 1).

Der aus der Subklonierung des SK-HEP-1-Klons #1963 hervorgegangene Zellklon #5235 wurde bei der ECACC hinterlegt und hat die amtliche provisorische Kenn-Nummer 94 092111.

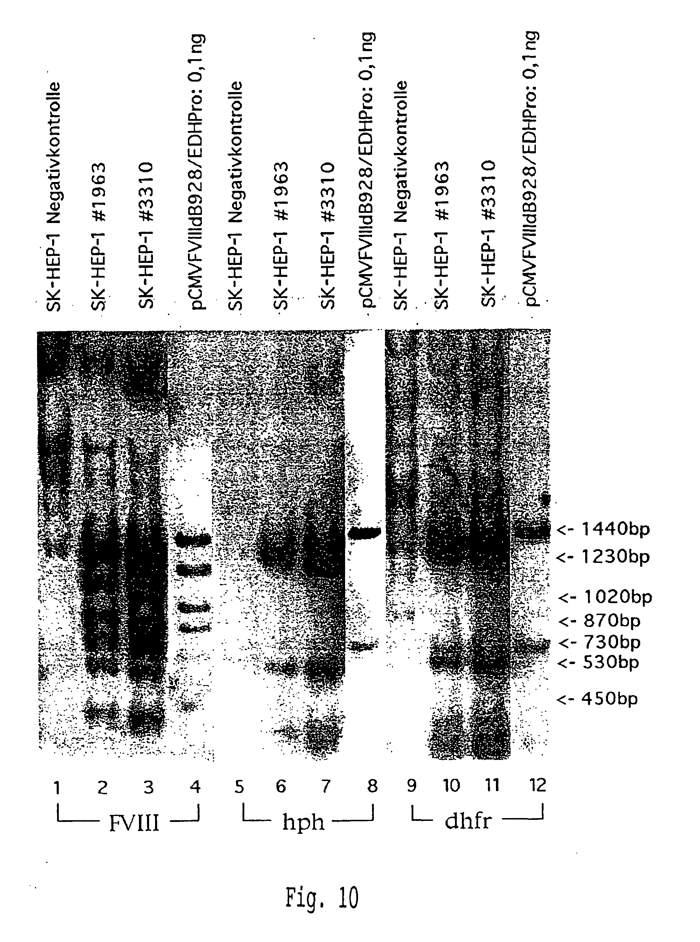

Expression von Faktor VIII: Expression von FVIIIdB928: Der exprimierte FVIIIdB928 wurde in der Western Blot-Analyse überprüft (Fig. 10). Die Zahlenwerte am Rand geben das Molekulargewicht in kDa an. Zusätzlich ist die gemessene Faktor VIII-Aktivität in milli Units (mU) angegeben. Dabei konnte gezeigt werden, daß das Faktor VIII-spezifische Bandenspektrum auftrat, mit Ausnahme einer Bande bei rund 140kDa. Der von 293 als auch SK-HEP-1-Zellen exprimierte FVIIIdB928 weist die typischen Banden auf, die im Zuge der Aktivierung von Faktor VIII auftreten. FVIIIdB928 exprimiert von 293-Zellen (Spur 1 und 2) unterschied sich insofern von Faktor VIII aus SK-HEP-1-Zellen (Spur 5 und 6), als in größerem Ausmaß die Banden bei 50, ,45 und 43 kDa nachgewiesen werden konnten.

Die Expression von FVIIIdB928 und komplettem Faktor VIII in 293- und SK-HEP-1-Zellen ist in Tabelle 2 zusammengefaßt. 293-Zellen exprimierten initial 100-200mU FVIIIdB928/10⁶-Zellen und 24h, diese Werte ließen sich nach Subklonierung weiter steigern.

**Tabelle 2**

| | 293-Zellen/ EDH-System mU/10⁶-Zellen | SK-HEP-1-Zellen/EDH-System mU/10⁶-Zellen | CHO-Zellen (Dorner et al. JCB 105; 2666 (1987); Kaufman et al., 1988) mU/ml |
|---|---|---|---|
| Initialselektion | FVIIIdB928: 100 - 200 | FVIIIdB928: 300 - 1000 | FVIIIdB: nicht gezeigt |
| | FVIIIc: 5-10 | FVIIIc: 5-10 | FVIIIc: 0,1 |
| Amplifikation | / | / | / |
| 1500µg HyB | / | FVIIIdB928: 1000 - 3000 | / |
| 1 µM MTX | / | / | FVIIIdB:1000-2000 |
| | | | |
| 1 mM MTX +vWF | | | FVIIIc: 1000 |

Die FVIIIdB928 transfizierten SK-HEP-1-Zellen zeigten eine initiale Expression von 300mU FVIIIdB928/10⁶-Zellen und 24h, nach Subklonierung stieg dieser Wert auf 500-1000mU FVIIIdB928/10⁶-Zellen und 24h. Die Amplifikation ausgehend von 200µg HyB auf 1500µg HyB führte zu einer Expressionssteigerung bis zu 3000mU FVIIIdB928/-10⁶-Zellen und 24h. Die Amplifikation über den DHFR-Anteil des EDH-Markers erfolgte wie bei Beispiel 1 beschrieben, da die hier dargestellten Zellklone noch über das Potential der mit der üblichen DHFR-Amplifikation einhergehenden Expressionssteigerung verfügten.

Expression von komplettem Faktor VIII: Die FVIIIc-transfizierten 293 und SK-HEP-1-Zellen wiesen unter HyB-Selektion eine maximale Expression von 10mU FVIIIC/10⁶-Zellen und 24h auf. Die weitere Amplifikation erfolgte wie oben beschrieben.

Die mit dem hier beschriebenen System erzielten Expressionswerte sind vor allem in dem Zusammenhang mit den in der Literatur erreichten Expressionsdaten zu beurteilen. Die hier bechriebenen FVIIIc/SK-HEP-1-Zellen exprimierten bereits initial 10mU FVIIIC/10⁶-Zellen und 24h. Der Vergleich der in der Literatur beschriebenen Expressionen von bekannten, B-Domänen deletierten Faktor VIII-Konstrukten mit dem hier beschriebenen System fällt ähnlich aus. Im hier beschriebenen System der Expression von FVIIIdB928/EDHPro in SK-HEP-1-Zellen ließen sich ohne MTX-Amplifikation bereits 3U FVIIIdB928/10⁶-Zellen und 24h nachweisen. Dieser Wert ist vor allem unter Berücksichtigung des noch nicht genutzten DHFR-Amplifikationspotentials zu beurteilen, welches sich wie bei Kaufman et al., 1988, supra beschrieben für bis zu 10000facher Expressionssteigerung nutzen ließ. Zusätzlich eröffnet gemäß Kaufman et al, 1988, die Möglichkeit der vWF-Coexpresion eine weitere Steigerung der Faktor VIII-Ausbeute.

Zusammenfassend läßt sich die Expression von Faktor VIII in CHO der in humanen Leberzellen wie SK-HEP-1 Zellen wie in Tabelle 3 gegenüberstellen.

**Tabelle 3**

| CHO-Zellen als FVIII-Expressionssystem | SK-HEP-1-Zellen als FVIII-Expressionssystem |
|---|---|
| - nach initialer Selektion nicht nachweisbare Expression von B-Domänen deletiertem FVIII und FVIIIc - "blinde" Amplifikation notwendig - dadurch sehr großer "Screening"-Aufwand - aufgrund der geringen FVIII-Expression ist sehr hohe Amplifikation erforderlich, die sehr zeitaufwendig ist - die hohe Amplifikation erfordert mehr "Screening" - CHO-Zellen verfügen über "double minute" Chromosomen, die mit instabiler Fremdprotein-Expression verbunden sind - hohe Amplifikation bewirkt ein größeres Maß an genetischer und expressionsbezogener Instabilität - Unterschiede der posttranslationalen Modifikationen von Fremdproteinen (z.B. Glykosylierungen) im Vergleich zu humanen Proteinen - mögliche Unterschiede des FVIII, da er in Ovarienzellen exprimiert wurde | hohe FVIIIdB928 und FVIIIc-Expression nach initialer Selektion -dadurch gezielte Amplifikation derjenigen Zellklone, die initial die größte Menge an FVIII exprimieren -damit verbunden wesentlich geringerer "Screening"-Aufwand -Zeitersparnis aufgrund der rascheren Herstellung hoch FVIII exprimierender Zellinien -aufgrund der initial relativ hohen Expression von FVIII ist geringere Amplifikation ausreichend -dadurch verringert sich das "Screening"-Ausmaß -eine geringere Anzahl an Gen-Kopien läßt sich leichter stabilisieren -keine Spezies abhängigen Veränderungen der posttranslationalen Modifikationen wie z.B. Glykosylierungen -authentischer FVIII, da er in einer Leberzellinie exprimiert wurde |

### Beispiel 3: Expression des von Willebrand-Faktors in transformierten Zellen unter spezieller Berücksichtigung von humanen Leberzellinien.

Von Willebrand Faktor (vWF) kommt eine bedeutende Rolle im Rahmen seiner Faktor VIII stabilisierenden Funktion zu. Aus diesem Grund war einerseits die Coexpression von vWF zusammen mit Faktor VIII interessant, andererseits war auch die Expression von vWF allein bedeutend.

### Konstruktion der Plasmide:

pAct/MCS: pActin beinhaltet den 3.3kb großen Promoter des menschlichen β Actin-Gens sowie 1kb der 5' UTR des β Actin-Gens. Die 5' UTR enthält das erste Intron des β Actin-Gens. Es schließt sich eine MCS an, gefolgt von der SV40-Polyadenylierungsstelle. pActin basiert auf Plasmid pSVβ (MacGregor und Caskey, supra, siehe Beispiel 1). Aus dem resultierenden Plasmid pSVMCS wurde das den SV40-Promoter/Enhancer und das SV40 16/19S-Intron enthaltende EcoRI-SalI-Fragment entfernt; stattdessen wurde das Actin-Promoter und 5' UTR Actin-Intron enthaltende EcoRI-SalI-Fragment aus pHβAPr-1 (Gunning et al., PNAS 84: 4831, 1987) eingesetzt. Dieses Plasmid wurde pActin genannt. Dieses Plasmid wurde mit ClaI und SalI geschnitten und mit den Oligonucleotiden #1293: 5' TCG ATG TTA ACT ACG TAG CTA GCG CGG CCG CCG TAC GTC GCG AGT CGA CAA TAT TGA TAT CGG TAC CGG TAC CAC TAG TGT 3' (SEQ.ID.No. 22) und #1294: 5' CGA CAC TAG TGG TAC CGG TAC CGA TAT CAA TAT TGT CGA CTC GCG ACG TAC GGC GGC CGC GCT AGC TAC GTA GTT AAC A 3' (SEQ.ID.No. 23) ligiert. Daraus entstand das Konstrukt pAct/MCS.

pAct/EDHPro: pAct/MCS wurde mit EcoRV geschnitten und das 2200bp große EDHPro-Fragment als SmaI und BglII, Pol. K. behandeltes Fragment aus pCMV/EDHPro eingesetzt, sodaß das Plasmid pAct/EDHPro entstand.

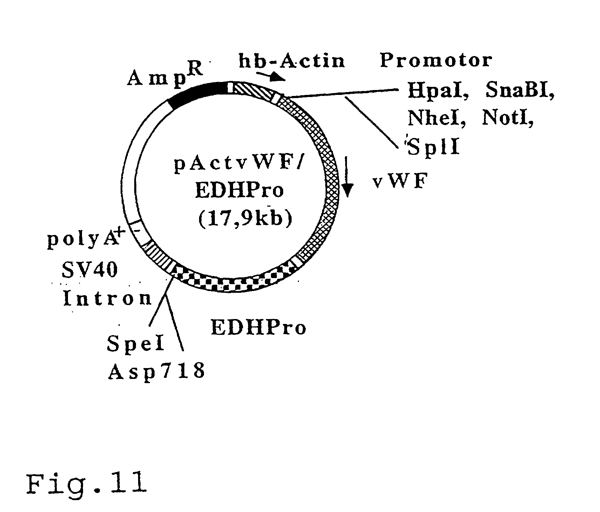

pActvWF/EDHPro: Ein aus ph-Act-vWF (Fischer et al., FEBS Letters 351; 345 (1994)) ausgeschnittenem EcoRI-Fragment, das die gesamte cDNA des menschlichen vWF, sowie etwa 200bp 5' und 130bp 3' UTR enthält, wird mit Pol. K. aufgefüllt und in die NruI-Schnittstelle von pAct/EDHPro eingesetzt. Daraus resultierte das Plasmid pActvWF/EDHPro (Fig. 11).

Abgesehen vom gesamten kodierenden Bereich des vWF enthält dieses Fragment 200bp der untranslatierten (UTR) 5'-Region und 150bp der untranslatierten 3'-Region.

Herstellung der permanenten Zellinien: Initialselektion und Amplifikation erfolgten wie bei Beispiel 2 beschrieben.

vWF-Quantifizierung mittels ELISA: Die vWF-Quantifizierung erfolgte mittels des von Boehringer Mannheim, BRD erhältlichen ELISA-Systems ("Aserachrom vWF, Nr. 136 0272).

Proteinnachweis mittels Western Blot-Analysen: Die Western Blot-Analysen wurden entsprechend den Beschreibungen in Beispiel 2 durchgeführt. Als erster Antikörper wurde ein polyklonaler Kaninchen-Anti-vWF-Antikörper (Firma Dakopatts, Dänemark) in einer Verdünnung von 1:100 eingesetzt. Als zweiter Antikörper wurde ein Ziege-Anti-Kaninchen-Antikörper (Firma BioRad, CA, USA) in einer Verdünnung von 1:7500 verwendet.

Untersuchungen der DNA- und RNA-Struktur: Die Präparationen von DNA und RNA erfolgten wie in Beispiel 1 beschrieben. Zur Hybridisierung im Rahmen der Southern Blot-Analysen bzw. Northern Blot-Analysen wurden vWF-, dhfr- und hph-Fragmente aus den jeweiligen Plasmiden isoliert (also aus pActvWF/EDHPro, pSVDHFR, pCMV/Hy).

Herstellung und Analyse von pActvWF/EDHPro-transfizierten Zellinien: Analog den Beschreibungen bei Beispiel 2 wurden 293- und SK-HEP-1-Zellen mit dem Expressionsplasmid pActvWF/EDHPro transfiziert und stabil vWF-exprimierende Zellinien selektiert und im Rahmen von Southern Blot-Analysen charakterisiert. Im Anschluß an die Selektion mit HyB wurden sowohl 293- als auch SK-HEP-1-Zellen ausgehend von 100nM MTX über die dhfr-Einheit des EDH-Markers amplifiziert. In beiden Fällen wurde vWF in großen Mengen exprimiert. Die Identität des exprimierten vWF mit plasmatischem vWF wurde mittels Western Blot-Analysen festgestellt. Die vWF-Quantifizierung erfolgte mittels ELISA-Bestimmungen. Zusätzlich wurde die Ristocetin-induzierte Trombozyten-Aggregation mittels des entsprechenden Tests der Behringwerke (OUBD, von Willebrand Reagenz) untersucht.

### Beispiel 4: Expression von rekombinantem, humanen Faktor IX in SK-HEP-1- und 293-Zellen

Aus einer "randomly primed" humanen Leber Lambda gt10 Phagen-Bibliothek wurde die cDNA des humanen Faktor IX isoliert. Das Faktor IX cDNA-Fragment umfaßt neben der kodierenden Region 4 Nukleotide der 5' UTR und 48 Nukleotide der 3' UTR. Dieses 1,4kb große, von EcoRI-Linkern flankierte Fragment wurde anschließend in die EcoRI-Stelle des Plasmids Bluescript II KS- (Strategene) gesetzt. Dieses Plasmid wurde pBlueII KS- FIX genannt.

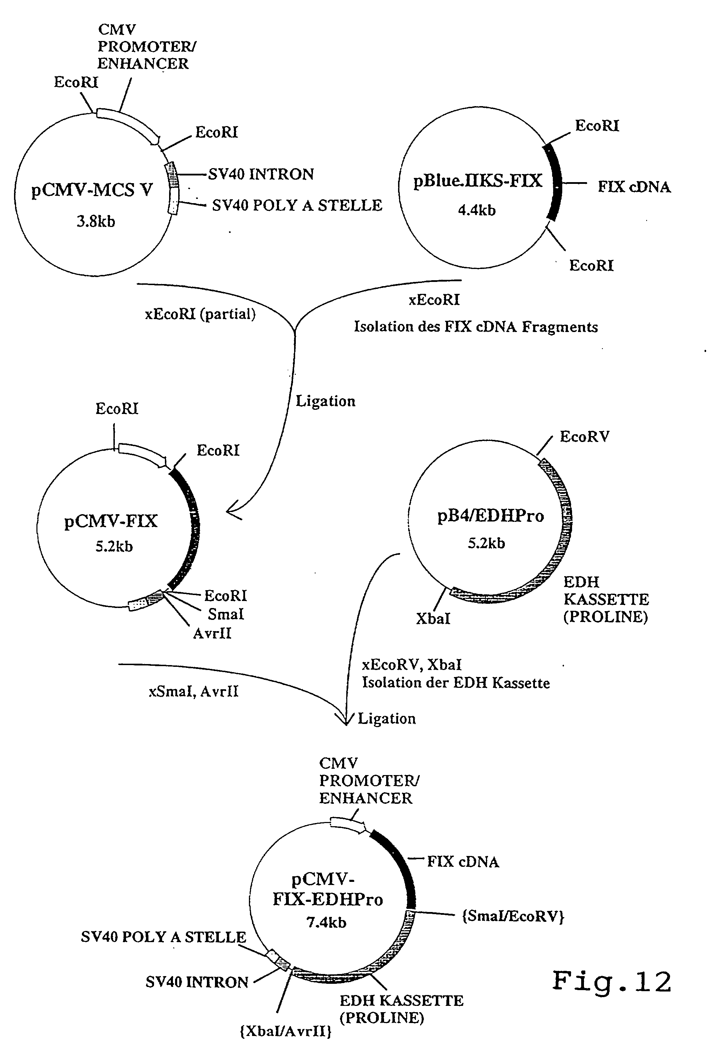

Wie in Fig. 12 schematisch beschrieben, wird mittels Standard-Klonierungs-Methoden (Maniatis et al., supra) die Faktor IX-cDNA als EcoRI-Fragment in das ebenfalls EcoRI-geschnittene Plasmid pCMV-MCS V gesetzt und ergibt pCMV-FIX. pCMV-MCS-V ist ein Folgeplasmid von pCMV-MCS (siehe Beispiel 1); in dessen XhoI-Stelle wurde die MCS mit der Sequenz 5'-TCGAATCGA TTGAATTCCC CGGGGTCCTC TAGAGTCGAC CTGCAGAAGC TTAGTACTAG TAGGCCTAGG GCCCTATCGA-3' (SEQ.ID.No. 24) eingefügt.

Das resultierende Plasmid pCMV-FIX wurde mit SmaI und AvrII geöffnet und die EDH-Kassette aus Plasmid pB4/EDHPro als EcoRV/XbaI-Fragment eingesetzt. Das resultierende Plasmid ist pCMV-FIX-EDHPro.

pB4/EDHPro: Die EDH-Kassette wurde als SmaI, BglIII-Fragment aus pCMV/EDHPro isoliert und in den Sma-, BamHI-geschnittenen Vektor pBluescript II SK- (Pharmacia, Schweden) eingesetzt.

293-(ATCC, CRL 1573) und SK-HEP-1-(ATCC, HTB 52)-Zellen, die routinemäßig in DMEM/Ham's F12-Medium supplementiert mit 2mM Glutamin und 10% fötalem Kälberserum wachsen, wurden mittels CaPO₄-Methode bzw. Elektroporation (BioRad Gene Pulser) zur Aufnahme von pCMV-FIX-EDHPro gebracht. Zwei Tage nach DNA-Aufnahme wurden die Zellen in unterschiedlichen Zelldichten ausplattiert, und das Medium zur Selektion mit 100µg (293) bzw. 200µg (SK-HEP-1) Hygromycin B/ml versehen. Zwei Wochen später wurden die resultierenden Zellklone isoliert und in separaten Zellkultur-Schalen zur Konfluenz gewachsen. In serumfreien 24 Stunden-Zellkulturüberständen, die mit 10µg Vitamin K₁/ml supplementiert sind, wurden anschließend Antigenmenge (ELISA), Funktionalität (entsprechende Aktivitätstests) und qualitative Integrität (Western Blot-Analyse) des sekretierten, rekombinanten Proteins untersucht. Die Zellzahl wurde nach Trypsinieren der Zellen (im Zellzahlmeßgerät der Fa. Schärfe, Reutlingen, Deutschland) bestimmt.

Zur Faktor IX-Antigen-Bestimmung wurde der Test-Kit der Fa. Boehringer Mannheim (Asserachrom Factor FIX-Ag, Diagnostica Stago) verwendet, wobei zur Standardkurven-Erstellung ein Referenzplasma (das IMMUNO Referenzplasma 5220005) verwendet wurde.

Zum Nachweis der Gerinnungsaktivität wurde ein Ein-Stufen- Gerinnungstest unter Verwendung eines Amelung KC10-Coagulometers eingesetzt. Hierbei wurden zunächst jeweils gleiche Teile der zu bestimmenden Probe, Faktor IX-Mangelplasma und Phospholipid/Kaolin-Aktivator-Lösung bei 37°C 4 min. inkubiert, anschließend zum Start der Reaktion ein Teil 25mmol CaCl₂ addiert, die Gerinnungszeit gemessen, und mit einer mittels eines Faktor IX-Standards erstellten Standardkurve ermittelt.

Zur Western Blot-Analyse wurden 10µl Zellkulturüberstand reduziert und denaturiert, und in denaturierenden 4% Sammel-/8%-Trenngelen nach Lämmli (Nature 227: 680, 1970) mit dem BioRad Mini-Protean II Dual Slab Gel-System aufgetrennt (BioRad Laboratories, Richmond, CA, USA). Die Proteine wurden nach erfolgtem Gellauf mit dem BioRad Mini Trans-Blot-System (BioRad Laboratories, Richmond, CA, USA) in Transferpuffer (25mM Tris, 192mM Glycin) auf Nitrozellulose-Membranen transferiert. Zur Visualisierung des rekombinanten Proteins wurde das Protoblot-System der Fa. Promega (Madison, WIS, USA) verwendet. Als Antikörper zur Faktor IX-Bindung wurde Kaninchen-Anti-Faktor IX-Serum der Fa. Dakopatts (Glostrup, Dänemark) eingesetzt.

Aktivitäts- und Antigen-Ausbeuten typischer Zellklone und zugehöriger Negativ-Kontrollen sind in Tabelle 4 aufgeführt.

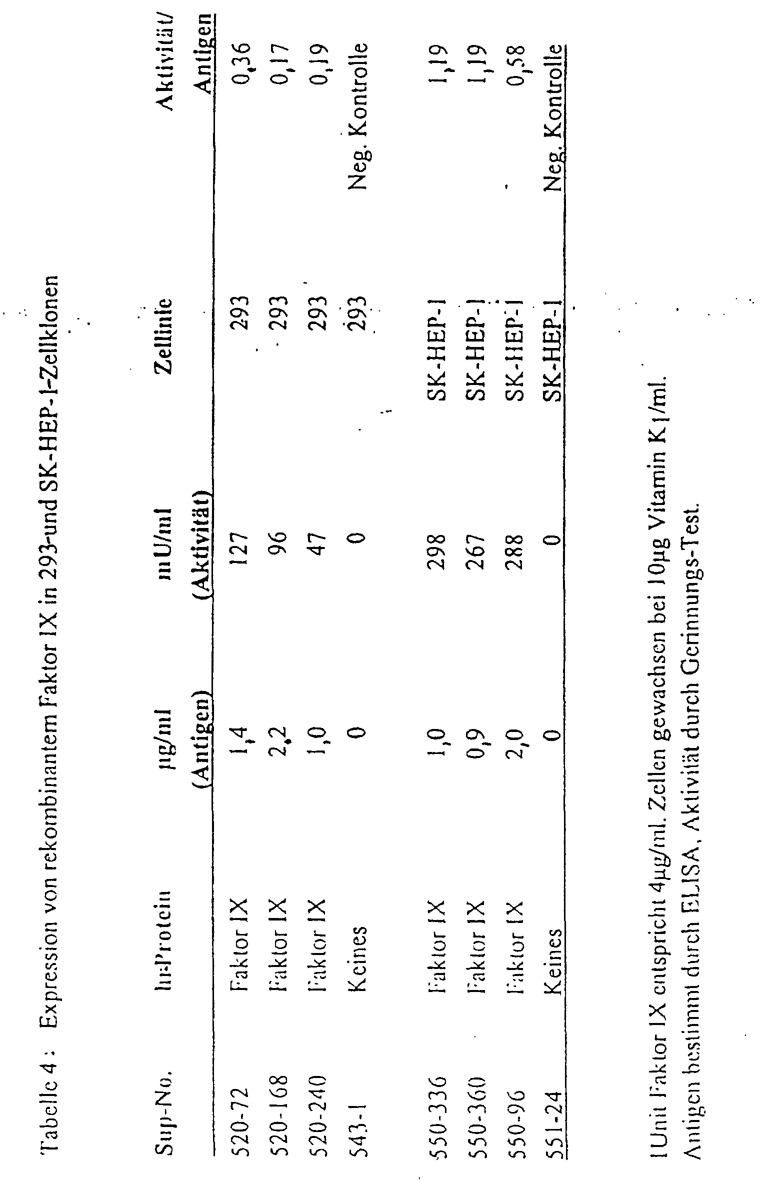

Prinzipiell läßt sich aus den Expressionsdaten schließen, daß mit dem erfindungsgemäßen Expressionssystem im Vergleich zu dem in der Literatur beschriebenen CHO-Expressionssystem bereits bei nicht-amplifizierten initialen Zellklonen erheblich höhere Expressionswerte von rekombinantem Faktor IX in SK-HEP-1- und 293-Zellen erzielen lassen, und der Anteil von funktionellem Faktor IX am Gesamt-Faktor IX wesentlich größer ist. Ein weiterer Vorteil des hier beschriebenen Selektionssystems besteht darin, daß von allen nach Transfektion/Elektroporation isolierten Einzelklonen alle (>95%) auch rekombinanten Faktor IX produzieren; dies steht im krassen Gegensatz zu dem herkömmlichen CHO-dhfr-Expressionssystem, in dem sowohl bei Cotransfektion als auch bei Verwendung bicistronischer mRNAs ohne interne Ribosomen-Bindungsstellen nur ein Bruchteil der isolierten Klone Faktor IX produzieren (Ehrlich et al., JBC 264: 14298, 1989).

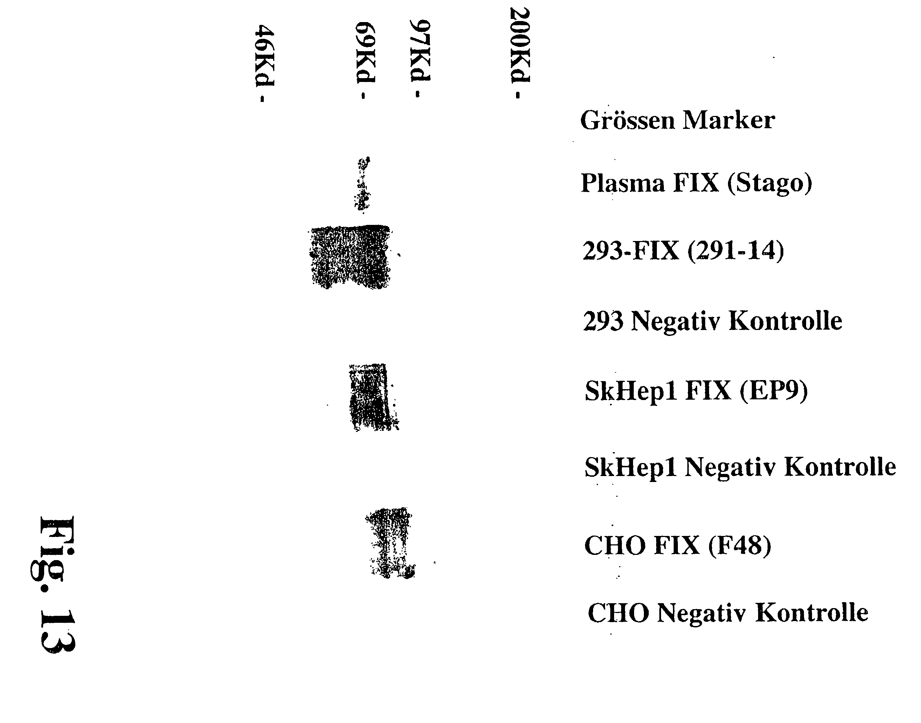

Fig. 13 zeigt den Western Blot von rekombinantem Faktor IX aus repräsentativen 293- und SK-HEP-1-Zell-Klonen im Vergleich zu plasmatischem Faktor IX und rekombinantem Faktor IX aus CHO-Zellen.

Rekombinanter Faktor IX aus allen drei Zellinien zeigt ein dem plasmatischen Faktor IX vergleichbares Molekulargewicht. 293-Faktor IX wurde vom 293-Klon 291-14 erhalten, SK-HEP-1-Faktor IX vom Zellklon EP 9. Als Kontrolle wurde auch rekombinanter Faktor IX aus dem CHO-Zellklon F 48, der mittels konventioneller Faktor IX/dhfr-Cotransfektion erstellt wurde, aufgetragen. 293-, SK-HEP-1- und CHO-Zellen, die keine Expressionsplasmide enthalten, produzieren keinen Faktor IX.

Bei den Expressionsdaten sei noch im speziellen darauf hingewiesen, daß das Amplifikationspotential im vorliegenden Beispiel noch nicht ausgenutzt wurde. Die Ausbeuten lassen sich nach erfolgter Amplifikation noch drastisch steigern.

### Beispiel 5: Expression von rekombinantem, humanen Protein C in SK-HEP-1- und 293-Zellen

Aus einer "randomly primed" humanen Leberzell-λgt10-Phagen-Bibliothek wurde die cDNA des humanen Protein C isoliert. Neben der kodierenden Region enthält die cDNA auch 100bp der untranslatierten (UTR) 5'-Region und 500bp der untranslatierten 3'-Region, und ist beiderseits von EcoRI-Schnittstellen flankiert. Dieses 1,9 kb große Fragment wurde in die EcoRI-Stelle des Plasmids pUC13 (Pharmacia) eingesetzt und pPrtC-1 genannt.

pPrtC-1 enthält auf Aminosäureebene im Vergleich zur publizierten Protein C-Sequenz (Beckman et al., NAR 13:5233, 1985; Foster und Davie, PNAS 81:4766, 1984) zwei Unterschiede: Codon 76 des reifen Protein C enthält statt der publizierten Sequenz TTC das Triplett CTC (dies resultiert in einem Aminosäureaustausch von PHE zu LEU); zum anderen weist pPrtC-1 eine in-frame-Deletion jener 5 Codons (5'-GGC GAC AGT GGG GGG-3') auf, die für die Aminosäuren 358 bis 362 (GLY-ASP-SER-GLY-GLY) des reifen Protein C kodieren.

Mittels Standard-Klonierungs-Methoden (Maniatis et al., supra) wurde mit PstI ein 1.5kb großes Protein C-Fragment (welches den 5' UTR, jedoch nur noch 15bp des 3' UTR-Bereichs enthält) aus pPrtC-1 ausgeschnitten und in den PstI geöffneten pTM3 (Moss et al., Nature 348:91, 1990) inseriert; das resultierende Plasmid ist pTM3-PrtC.

Unter Verwendung eines Mutagenese-Sets (Sculptor In Vitro Mutagenesis Kits (Firma Amersham) und des DNA-Primers 5'-TGTGAGCTGCCCCATGGTGGAGGCACTGGC 3' (SEQ.ID.No. 25) wurde die mit dem Translationsinitiationscodon ATG überlappende DNA-Sequenz in pTM3-PrtC in eine NcoI-Schnittstelle umgewandelt. Das resultierende Plasmid wurde NcoI geschnitten, und religiert, um die in pTM3 gelegene NcoI-Schnittstelle an die neu geschaffene NcoI Schnittstelle am 5'-Ende des kodierenden Bereiches der PrtC-cDNA zu fusionieren. Hierdurch ging der gesamte 5' UTR der ProtC-cDNA verloren.

Mit Hilfe des Sculptor In Vitro Mutagenesis Kits und des Primers 5'-GTGGAAGGAGGCGACCATGGGCCCCCCACTGTCGCCCTCGCAGGCATCCTGCCGGTC-3' (SEQ.ID.No. 26) wurden zunächst die fehlenden 15 Nukleotide wieder in pTM3-PrtC inseriert, um die o.g. Deletion zu reparieren. Das resultierende Plasmid wurde pTM3-PrtCpt. mut. genannt.

Auf analoge Art wurde schließlich mit dem Primer 5'-GCAGTCGCAGCTGAAGCTGCCGAT-3' (SEQ.ID.No. 27) in pTM3-PrtCpt. mut. die Punktmutation in Codon 76 in die Wildtyp-Sequenz verändert. Das resultierende Plasmid wurde pTM3-PrtCwt. genannt.

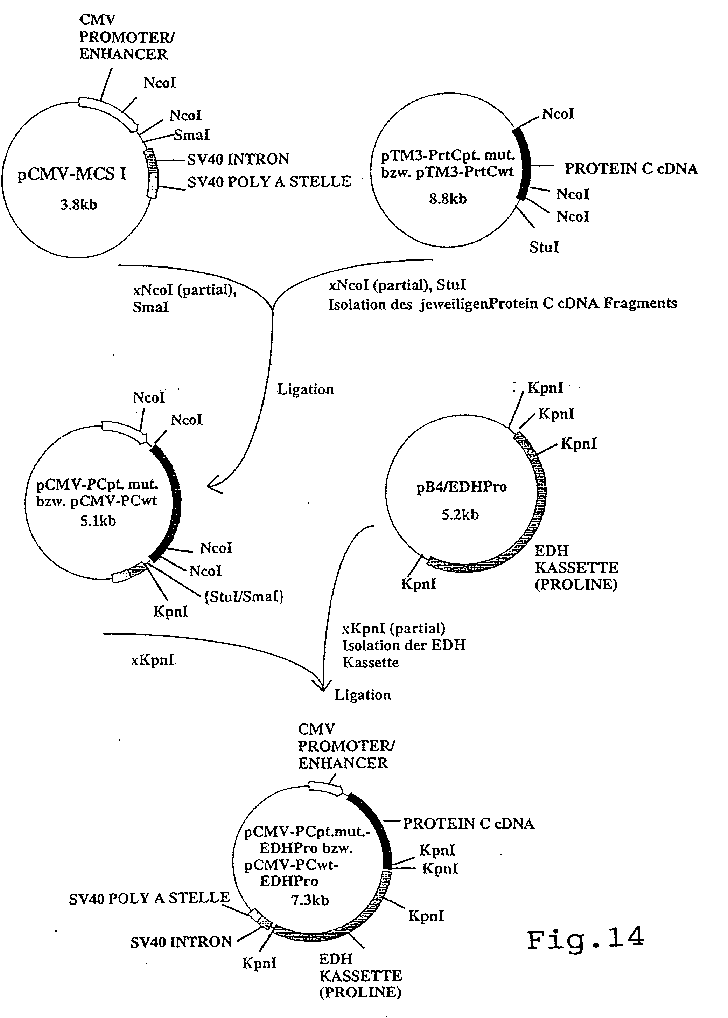

Wie in Fig. 14 schematisch beschrieben, wurden die PCwt bzw. PCpt. mut. cDNA-Fragmente aus pTM3-PCwt. bzw. pTM3-PCpt. mut. als NcoI, StuI-Fragment in das NcoI, SmaI geschnittene Plasmid pCMV-MCS I gesetzt. pCMV I ist ein Abkömmling des Plasmids pCMV-MCS. Dieses Plasmid beinhaltet den "Immediate Early Gene"-Promoter/Enhancer des menschlichen Cytomegalovirus und 80bp der 5' UTR des zugehörigen Gens. Es schließt sich die MCS mit der Sequenz 5'-TCGACCATGGAAGCTTATCGATCCCGGGAA TTCGGTACCG TCGACCTTGCA GGTGCACGGG CCCAGATCTG ACTGATCGA-3' (SEQ.ID.No. 28) an, gefolgt von dem SV40 16S/19S-Intron und der SV40-Polyadenylierungsstelle.

Die resultierenden Plasmide pCMV-PCwt bzw. pCMV-PCpt. mut. wurden mit KpnI geöffnet und die EDH-Kassette aus Plasmid pB4/EDHPro (siehe Beispiel 4) als KpnI-Fragment eingesetzt. Die resultierenden Plasmide sind pCMV-PCwt-EDHPro bzw. pCMV-PCpt. mut.-EDHPro.

Beide Plasmide wurden, wie in Beispiel 4 beschrieben, in 293-(ATCC, CRL 1573) und SK-HEP-1-(ATCC, HTB 52)-Zellen eingeschleust und Zellklone isoliert.

In serumfreien 24 Stunden-Zellkulturüberständen, die mit 10µg Vitamin K₁/ml supplementiert sind, wurden anschließend Antigenmenge (ELISA), Funktionalität (entsprechende Aktivitätstests) und qualitative Integrität (Western Blot-Analyse) des sekretierten, rekombinanten Proteins untersucht. Die Zellzahl wurde nach Trypsinieren der Zellen (im Zellzahlmeßgerät der Fa. Schärfe, Reutlingen, Deutschland) bestimmt.

Zur Protein C-Antigen-Bestimmung wurde ein Test-Kit (Asserachrom Factor Protein C-Ag, Diagnostica Stago, Fa. Boehringer Mannheim) verwendet, wobei zur Standardkurven-Erstellung ein mitgelieferter Standard benutzt wurde.

Zum Nachweis der Gerinnungsaktivität wurde ein Ein-Stufen-Gerinnungstest unter Verwendung eines Amelung KC4-Coagulometers eingesetzt. Hierbei wurden zunächst jeweils gleiche Teile der zu bestimmenden Probe, Protein C-Mangelplasma, Protac und Phospholipid/Kaolin-Aktivator-Lösung bei 37°C 4 min. inkubiert, anschließend zum Start der Reaktion ein Teil 25mmol CaCl₂ addiert, die Gerinnungszeit gemessen, und mit einer mittels eines Protein C-Standards erstellten Standardkurve ermittelt.

Zur Western Blot-Analyse wurden 10µl Zellkulturüberstand reduziert und denaturiert, und in denaturierenden 4% Sammel-/10% Trenngelen nach Lämmli (Nature 227: 680, 1970) mit dem BioRad Mini-Protean II Dual Slab Gel-System aufgetrennt (BioRad Laboratories, Richmond, CA, USA). Die Proteine wurden nach erfolgtem Gellauf mit dem BioRad Mini Trans-Blot-System (BioRad Laboratories, Richmond, CA, USA) in Transferpuffer (25mM Tris, 192mM Glycin) auf Nitrozellulose-Membranen transferiert. Zur Visualisierung des rekombinanten Proteins wurde das Protoblot-System der Fa. Promega (Madison, WI, USA) verwandt. Als Antikörper zur Protein C-Bindung wurde Kaninchen-Anti-Protein C-Serum (Fa. Dakopatts; Glostrup, Dänemark) eingesetzt.

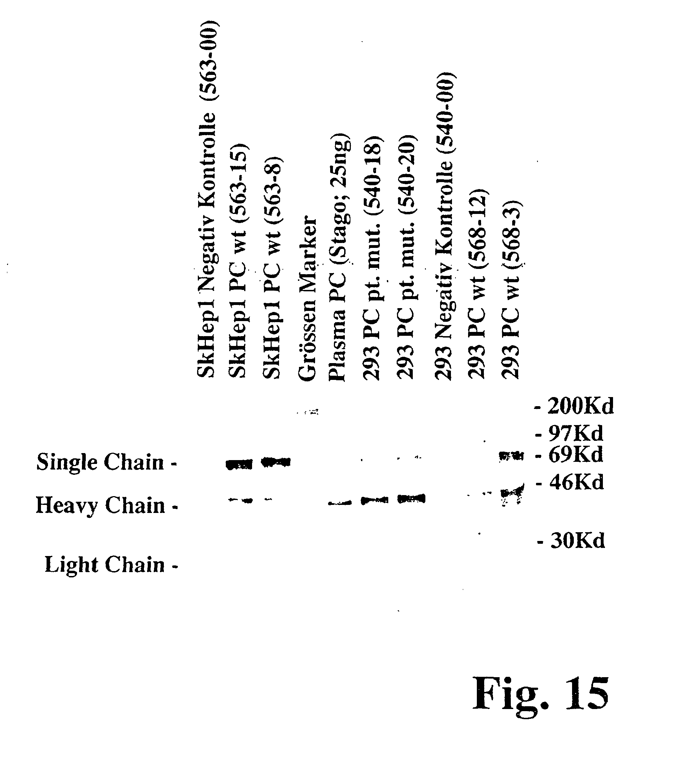

Aktivitäts- und Antigen-Ausbeuten typischer Zellklone und zugehöriger Negativ-Kontrollen sind in Tabelle 5 aufgeführt; Fig. 15 zeigt den Western Blot von rekombinanten Protein C aus 293- und SK-HEP-1-Zellen im Vergleich zu plasmatischem Faktor IX.

Während in nicht-transfizierten SkHepl-Zellen (Probe 563-00) kein Protein C nachweisbar ist, zeigen sowohl mit wt wie auch mit punktmutierter Protein C-cDNA transfizierte 293 bzw. SkHepl-Zellen entsprechende Expression. In allen Fällen sind schwere und leichte Ketten des Protein C nachweisbar, vergleichbar dem plasmatischen Protein C. Von 293 und SkHepl-Zellen produziertes wt-Protein C ist jedoch nur zu etwa 50% (Klone 568-12, 568-3) bzw. 30% (Klone 563-15, 563-8) in schwere und leichte Ketten prozessiert, während das verbleibende Material als unprozessiertes "single-chain"-Molekül vorliegt. Im Gegensatz dazu ist das punktmutierte Protein C zum überwiegenden Teil in schwere und leichte Ketten prozessiert, wie an den Überständen der beiden 293 Zellklone 540-18 und 540-20 ersichtlich ist. Die Molekulargewichte eines mit aufgetragenen Größenmarkers sind an der rechten Seite der Fig. 15 angegeben.

Der Artikel von Grinnell et al., Adv. Appl. Biotechnol. Series 11: 29-63, 1990, faßt die wt-Protein C-Expressionsdaten der Arbeitsgruppe bei Eli Lilly zusammen. Hieraus wird ersichtlich, daß bei initialselektierten, nicht-amplifizierten Zellklonen die maximal erreichten Expressionsdaten 1,15 µg/10⁶-Zellen und Tag nicht überschritten; bei dem von uns beschriebenen Expressionssystem sind im Gegensatz dazu durchaus 3-fach höhere Expressionsraten möglich, wie im Falle des Klons 568-12 demonstriert (Tabelle 5).

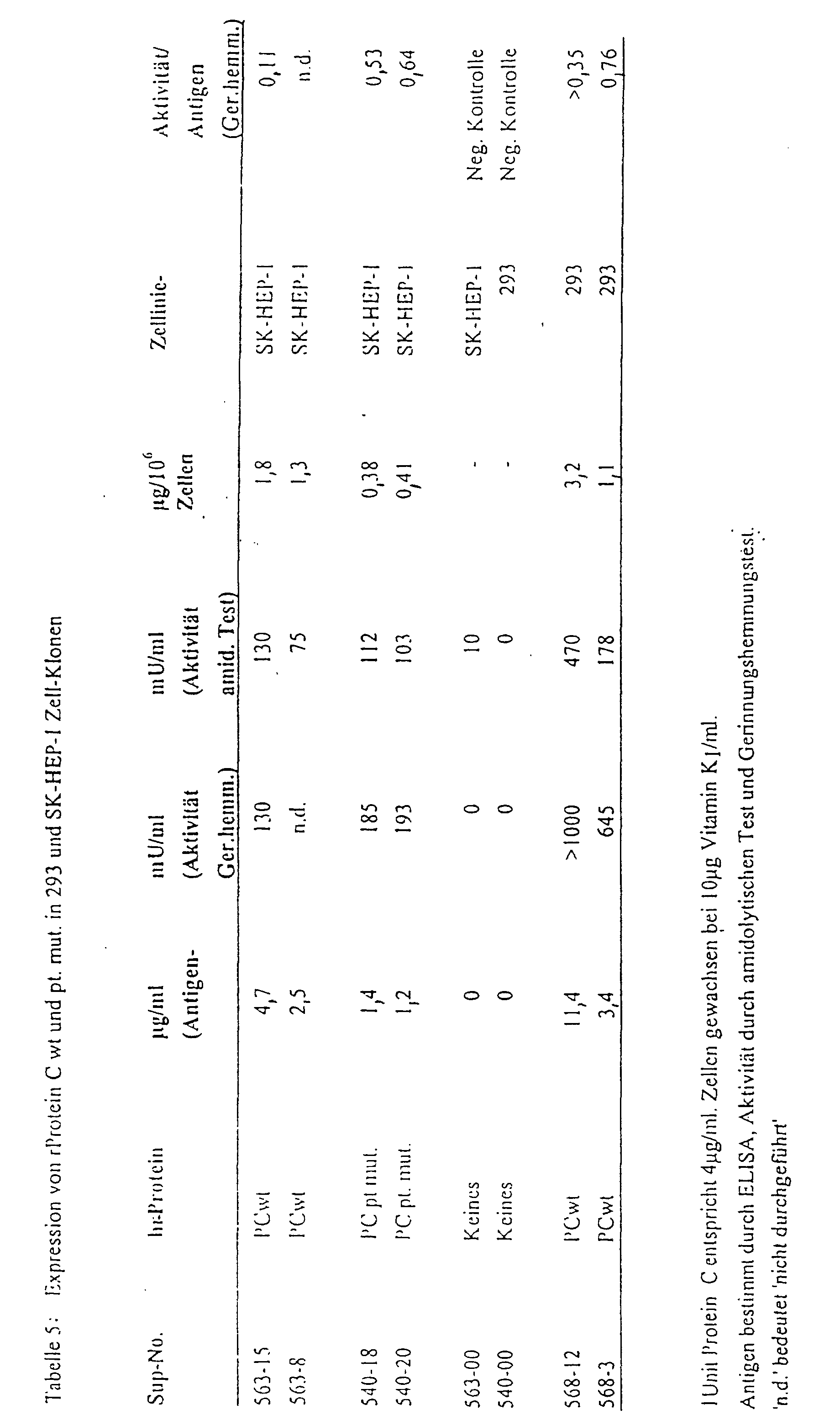

### Beispiel 6: Expression von humanem Serum Albumin (HSA) in transformierten SK-HEP-1-Zellen

### Konstruktion des HSA-Expressionsplasmids:

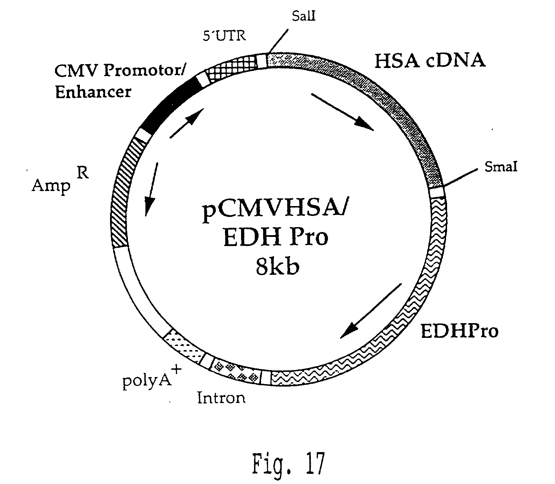

Das Expressionsplasmid pCMVFVIIIdB928/EDHPro (siehe Beispiel 2) wurde mit SmaI und SalI geschnitten, die FVIII-cDNA entfernt und mit der SmaI, SalI geschnittene HSA-cDNA aus pAlb4 ligiert. Dabei entstand das Expressionsplasmid pCMVHSA/EDHPro (Fig. 17).

Herstellung und Analyse von pCMVHSA/EDHPro-transfizierten Zellinien:

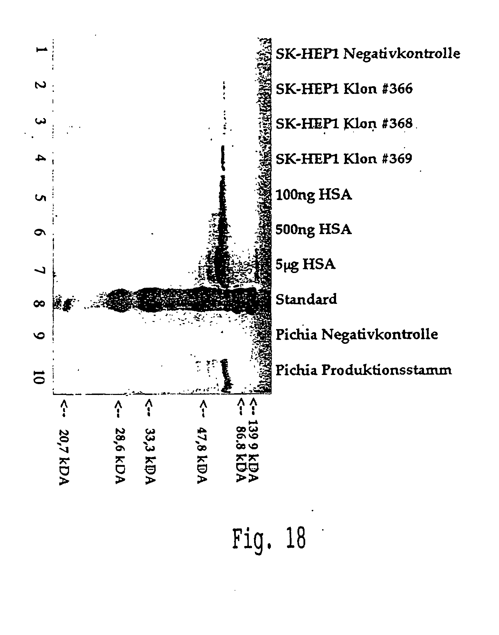

Analog den Beschreibungen bei Beispiel 2 wurden SK-HEP-1-Zellen mit dem Expressionsplasmid pCMVHSA/EDHPro transfiziert und stabil HSA-exprimierende Zellinien selektiert. Die Selektion erfolgte mit HyB beginnend bei 200 µg/ml und wurde in der Folge auf 400 µg/ml erhöht. Im Anschluß an die Selektion mit HyB wurden die SK-HEP-1-Zellen ausgehend von 100 nM MTX über die dhfr-Einheit des EDH-Markers amplifiziert. Auf der Stufe von 400 µg HyB konnten bis zu 1,7 µg HSA/10⁶-Zellen und 24 Stunden und 2,6 µg HSA/ml nachgewiesen werden. Die Identität des exprimierten HSA mit plasmatischem HSA und HSA aus Pichia pastoris wurde mittels Western Blot-Analysen festgestellt (Fig. 18). Die HSA-Quantifizierung erfolgte mittels ELISA-Bestimmungen.

### Materialien und Methoden

### Konstruktion der Plasmide:

pCMVHSA/EDHPro: pCMVFVIIIdB928/EDHPro wurde mit SmaI und SalI geschnitten, das FVIIIdB928-Fragment entfernt und stattdessen ligiert mit dem SmaI, SalI geschnittene HSA-Fragment aus pAlb4 (Fig. 17). pAlb4 setzt sich aus pBluescript 4 SK- und der HSA-cDNA (Lawn et al., Nucleic Acid Res. 9: 6103-6114, (1981); Dugaiczyk et al., PNAS 79: 71-75 (1982)) zusammen.

### Herstellung der permanenten Zellinien:

Initialselektion und Amplifikation erfolgten wie in Beispiel 2 beschrieben.

### HSA-Quantifizierung mittels ELISA:

Die HSA-Quantifizierung im ELISA erfolgte mittels des monoklonalen Anti-HSA-Antikörpers (Pierce) und des von Dakopatts, Dänemark, erhältlichen Kaninchen-Anti-HSA-Antikörperserums, welches direkt mit Peroxidase gekoppelt vorlag.

### Proteinnachweis mittels Western Blot-Analysen:

Die Western Blot-Analysen wurden entsprechend den Beschreibungen in Beispiel 2 durchgeführt. Als erster Antikörper wurde ein monoklonaler Anti-HSA-Antikörper (Monosan; Sanbio, Niederlande) in einer Verdünnung von 1:500 eingesetzt. Als zweiter Antikörper wurde ein Ziege-Anti-Maus-Antikörper (Firma BioRad, USA) in einer Verdünnung von 1:7500 verwendet.

## Patentansprüche

1. Expressionsplasmid, das eine dicistronische Transkriptions-/Translationseinheit enthält, welche eine Sequenz für ein Fremdprotein, eine interne Ribosomenbindungsstelle und eine Sequenz für ein Fusionsprotein umfasst, wobei das Fusionsprotein mindestens einen Selektions- und mindestens einen Amplifikationsmarker enthält, wobei der Amplifikationsmarker die Dihydrofolatreduktase ist.

2. Expressionsplasmid nach Anspruch 1, **dadurch gekennzeichnet, dass** die interne Ribosomenbindungsstelle die 5'-nicht-translatierte Region des Encephalomyocarditis-Virus (EMCV 5'UTR) ist.

3. Expressionsplasmid nach Anspruch 1 oder 2, **dadurch gekennzeichnet, dass** die kodierende Sequenz für das Fremdprotein 5' und die kodierte Sequenz für das Fusionsprotein 3' von der internen Ribosomenbindungsstelle liegt.

4. Expressionsplasmid nach einem der Ansprüche 1 bis 3, **dadurch gekennzeichnet, dass** es einen Promotor umfasst, der der CMV-Promotor, der SV 40-Promotor oder der humane β-Actin-Promotor ist.

5. Expressionsplasmid nach einem der Ansprüche 1 bis 4, **dadurch gekennzeichnet, dass** die dicistronische Transkriptions-/Translationseinheit zusätzlich ein Intron, vorzugsweise das Intron des SV40 t-Antigens, das 16s/19s-Intron oder das erste Intron des humanen β-Actin-Gens, und ein Poly-Adenylierungssignal, vorzugsweise das der frühen oder späten Transkriptionseinheit des SV40-Virus, enthält.

6. Expressionsplasmid nach einem der Ansprüche 1 bis 5, **dadurch gekennzeichnet, dass** die Sequenz für das Fusionsprotein aus zwei Teilsequenzen, nämlich dem Dihydrofolat Reduktase-Gen, und einem Selektionsmarkergen, vorzugsweise dem Hygromycin B-Phosphotransferase-Gen, besteht.

7. Expressionsplasmid nach einem der Ansprüche 1 bis 6, **dadurch gekennzeichnet, dass** das Selektions-/Amplifikationsmarker-Fusionsprotein bifunktionell ist und die für das Fusionsprotein kodierende Sequenz so konstruiert ist, dass der 5'- kodierenden Teilsequenz das Stopkodon und der 3'-kodierenden Teilsequenz gegebenenfalls das Startkodon fehlt.

8. Expressionsplasmid nach einem der Ansprüche 1 bis 6, **dadurch gekennzeichnet, dass** die kodierenden Sequenzen der beiden Proteinanteile des Fusionsproteins durch einen Spacer getrennt sind, insbesondere durch einen 15 Nukleotide langen Spacer.

9. Expressionsplasmid nach Anspruch 8, **dadurch gekennzeichnet, dass** die Spacersequenz für 5 Glycinreste kodiert und die Sequenz GGA GGC GGG GGT GGA (SEQ.ID.NO. 2) aufweist.

10. Expressionsplasmid nach Anspruch 8, **dadurch gekennzeichnet, dass** die Spacersequenz für 5 Prolinreste kodiert und die Sequenz CCA CCC CCG CCT CCA (SEQ.ID.NO. 1) aufweist.

11. Expressionsplasmid nach einem der Ansprüche 1 bis 10, **dadurch gekennzeichnet, dass** die Sequenz für das Fremdprotein eine Sequenz für ein humanes Plasmaprotein oder ein virales Protein bzw. ein Derivat oder ein Fragment davon umfasst.

12. Expressionsplasmid nach Anspruch 11, **dadurch gekennzeichnet, dass** die Sequenz für das Fremdprotein eine Sequenz für die humane Prothrombin-cDNA umfasst.

13. Expressionsplasmid nach Anspruch 11, **dadurch gekennzeichnet, dass** die Sequenz für das Fremdprotein eine Sequenz für die humane Faktor VIII-cDNA umfasst.

14. Expressionsplasmid nach Anspruch 11, **dadurch gekennzeichnet, dass** die Sequenz für das Fremdprotein eine Sequenz für die Deletionsmutante dB928 des humanen Faktor VIII umfasst.

15. Expressionsplasmid nach Anspruch 11, **dadurch gekennzeichnet, dass** die Sequenz für das Fremdprotein eine Sequenz für die humane Faktor IX-cDNA umfasst.

16. Expressionsplasmid nach Anspruch 11, **dadurch gekennzeichnet, dass** die Sequenz für das Fremdprotein eine Sequenz für die humane Protein C-cDNA umfasst.

17. Expressionsplasmid nach Anspruch 11, **dadurch gekennzeichnet, dass** die Sequenz für das Fremdprotein eine Sequenz für die humane von Willebrand Faktor-cDNA umfasst.

18. Expressionsplasmid nach einem der Ansprüche 1 bis 17, **dadurch gekennzeichnet, dass** es eine oder mehrere Expressionskassetten umfasst, welche die DNA Sequenzen SEQ.ID.NO. 6, SEQ.ID.NO. 7 oder SEQ.ID.NO. 8 enthalten.

19. Transfizierte eukaryontische Zelllinie, vorzugsweise ausgewählt aus den Zelllinien CHO, 293 oder humane LeberZelllinien, wie SK-HEP-1 oder Chang Liver, die mit einem Expressionsplasmid nach einem der Ansprüche 1 bis 18 transfiziert ist und ein Fremdprotein exprimiert.

20. Transfizierte eukaryontische Zelllinie nach Anspruch 19, **dadurch gekennzeichnet, dass** sie das humane Prothrombin exprimiert.

21. Transfizierte eukaryontische Zelllinie nach Anspruch 19, **dadurch gekennzeichnet, dass** sie den humanen Faktor VIII exprimiert.

22. Transfizierte eukaryontische Zelllinie nach Anspruch 19, **dadurch gekennzeichnet, dass** sie die Deletionsmutante dB928 des humanen Faktors VIII exprimiert.

23. Transfizierte eukaryontische Zelllinie nach Anspruch 19, **dadurch gekennzeichnet, dass** sie den humanen Faktor IX exprimiert.

24. Transfizierte eukaryontische Zelllinie nach Anspruch 19, **dadurch gekennzeichnet, dass** sie das humane Protein C exprimiert.

25. Transfizierte eukaryontische Zelllinie nach Anspruch 19, **dadurch gekennzeichnet, dass** sie den humanen von Willebrand Faktor exprimiert.

26. Verfahren zur Herstellung von Fremdproteinen, **dadurch gekennzeichnet, dass** eine eukaryontische Zelllinie mit einem Expressionsplasmid nach einem der Ansprüche 1 bis 18 transfiziert wird, die erhaltenen Klone durch einen Selektionsprozess unter der Kontrolle eines Selektionsmarkers isoliert und dabei vorzugsweise gleichzeitig amplifiziert werden, anschließend unter der Kontrolle des Amplifikationsmarkers eine weitere Amplifikation erfolgt, wobei das Fremdprotein exprimiert und geerntet wird.

27. Verfahren nach Anspruch 26, **dadurch gekennzeichnet, dass** der Selektionsprozess unter Verwendung von Hygromycin B und die weitere Amplifikation unter Verwendung von Methotrexat erfolgt.

28. Verfahren nach Anspruch 26 oder 27, **dadurch gekennzeichnet, dass** als Zelllinien CHO, 293 oder humane LeberZelllinien, wie SK-HEP-1 oder Chang Liver, mit einem Expressionsplasmid gemäß einem der Ansprüche 1 bis 18 transfiziert werden.

29. Verfahren nach einem der Ansprüche 26 bis 28, **dadurch gekennzeichnet, dass** rekombinante Blutgerinnungsfaktoren oder virale Proteine hergestellt werden.

30. Verfahren nach einem der Ansprüche 26 bis 29, **dadurch gekennzeichnet, dass** rekombinantes humanes Prothrombin, rekombinanter humaner Faktor VIII, rekombinanter humaner FVIIIdB928, rekombinanter humaner Faktor IX, rekombinantes humanes Protein C, rekombinantes humanes Serumalbumin oder rekombinanter humaner von Willebrand-Faktor hergestellt werden.

31. Verwendung von SK-Hep-1-Zellen, die mit einem Expressionsplasmid nach einem der Ansprüche 1 bis 18 transfiziert sind und ein Fremdprotein exprimieren, als Expressionsvehikel für Prothrombin, Faktor VIII, Faktor VIIIdB928, Faktor IX, Protein C, von Willebrand-Faktor und/oder Serumalbumin.

32. Expressionsplasmid nach Anspruch 11, **dadurch gekennzeichnet, dass** die Sequenz für das Fremdprotein eine Sequenz für die humane Serumalbumin-cDNA umfasst.

33. Transfizierte eukaryontische Zelllinie nach Anspruch 19, **dadurch gekennzeichnet, dass** sie das humane Serumalbumin exprimiert.

## Claims

1. An expression plasmid containing a dicistronic transcription/translation unit, which unit comprises a sequence for a foreign protein, an internal ribosome binding site, and a sequence for a fusion protein, the fusion protein containing at least one selection marker and at least one amplification marker, the amplification marker being dihydrofolate reductase.

2. An expression plasmid according to claim 1, **characterised in that** the internal ribosome binding site is the 5'-untranslated region of the encephalomyocarditis virus (EMCV 5'UTR).

3. An expression plasmid according to claim 1 or 2, **characterised in that** the sequence encoding the foreign protein is 5' and the sequence encoding the fusion protein is 3' from the internal ribosome binding site.

4. An expression plasmid according to any one of claims 1 to 3, **characterised in that** it comprises a promoter, which is the CMV promoter, the SV40 promoter or the human β-actin promoter.

5. An expression plasmid according to any one of claims 1 to 4, **characterised in that** the dicistronic transcription/translation unit additionally contains an intron, preferably the intron of the SV40 t-antigen, the 16s/19s-intron or the first intron of the human β-actin gene, and a poly-adenylating signal, preferably that of the early or late transcription unit of the SV40 virus.

6. An expression plasmid according to any one of claims 1 to 5, **characterised in that** the sequence for the fusion protein is comprised of two partial sequences, i.e. the dihydrofolate reductase gene, and a selection marker gene, preferably the hygromycin B phosphotransferase gene.

7. An expression plasmid according to any one of claims 1 to 6, **characterised in that** the selection/amplification marker fusion protein is bifunctional and that the sequence encoding the fusion protein is constructed such that the 5'-encoding partial sequence lacks the stop codon and the 3'-encoding partial sequence optionally lacks the start codon.

8. An expression plasmid according to any one of claims 1 to 6, **characterised in that** the encoding sequences of the two protein portions of the fusion protein are separated by a spacer, particularly by a spacer having a length of 15 nucleotides.

9. An expression plasmid according to claim 8, **characterised in that** the spacer sequence encodes 5 glycine residues and comprises the sequence GGA GGC GGG GGT GGA (SEQ.ID.No. 2).

10. An expression plasmid according to claim 8, **characterised in that** the spacer sequence encodes 5 proline residues and comprises the sequence CCA CCC CCG CCT CCA (SEQ.ID.No. 1).

11. An expression plasmid according to any one of claims 1 to 10, **characterised in that** the sequence for the foreign protein comprises a sequence for a human plasma protein or for a viral protein or for a derivative or fragment thereof, respectively.

12. An expression plasmid according to claim 11, **characterised in that** the sequence for the foreign protein comprises a sequence for human prothrombin cDNA.

13. An expression plasmid according to claim 11, **characterised in that** the sequence for the foreign protein comprises a sequence for human factor VIII cDNA.

14. An expression plasmid according to claim 11, **characterised in that** the sequence for the foreign protein comprises a sequence for the deletion mutant dB928 of human factor VIII.

15. An expression plasmid according to claim 11, **characterised in that** the sequence for the foreign protein comprises a sequence for human factor IX cDNA.

16. An expression plasmid according to claim 11, **characterised in that** the sequence for the foreign protein comprises a sequence for human protein C cDNA.

17. An expression plasmid according to claim 11, **characterised in that** the sequence for the foreign protein comprises a sequence for human von Willebrand factor cDNA.

18. An expression plasmid according to any one of claims 1 to 17, **characterised in that** it comprises one or several expression cassettes which contain the DNA sequences SEQ.ID.No. 6, SEQ.ID. No. 7 or SEQ.ID.No. 8.

19. A transfected eukaryotic cell line, preferably selected from the cell lines CHO, 293 or human liver cell lines, such as SK-HEP-1 or Chang liver, transfected with an expression plasmid according to any one of claims 1 to 18 and expressing a foreign protein.

20. A transfected eukaryotic cell line according to claim 19, **characterised in that** it expresses human prothrombin.

21. A transfected eukaryotic cell line according to claim 19, **characterised in that** it expresses human factor VIII.

22. A transfected eukaryotic cell line according to claim 19, **characterised in that** it expresses the deletion mutant dB928 of human factor VIII.

23. A transfected eukaryotic cell line according to claim 19, **characterised in that** it expresses human factor IX.

24. A transfected eukaryotic cell line according to claim 19, **characterised in that** it expresses human protein C.

25. A transfected eukaryotic cell line according to claim 19, **characterised in that** it expresses human von Willebrand factor.

26. A method of producing foreign proteins, **characterised in that** a eukaryotic cell line is transfected with an expression plasmid according to any one of claims 1 to 18, the clones obtained are isolated by a selection process under the control of a selection marker and therein are preferably simultaneously amplified, whereupon a further amplification is effected under the control of an amplification marker, wherein the foreign protein is expressed and harvested.

27. A method according to claim 26, **characterised in that** the selection process is effected by using hygromycin B and the further amplification is effected by using methotrexate.

28. A method according to claim 26 or 27, **characterised in that** CHO, 293 or human liver cell lines, such as SK-HEP-1 or Chang liver, are transfected as the cell lines with an expresison plasmid according to any one of claims 1 to 18.

29. A method according to any one of claims 26 to 28, **characterised in that** recombinant blood coagulation factors or viral proteins are produced.

30. A method according to any one of claims 26 to 29, **characterised in that** recombinant human prothrombin, recombinant human factor VIII, recombinant human FVIIIdB928, recombinant human factor IX, recombinant human protein C, recombinant human serum albumin or recombinant human von Willebrand factor are produced.

31. The use of SK-HEP-1 cells which have been transfected with an expression plasmid according to any one of claims 1 to 18 and which express a foreign protein, as an expression vehicle for prothrombin, factor VIII, factor VIII dB928, factor IX, protein C, von Willebrand factor and/or serum albumin.

32. An expression plasmid according to claim 11, **characterised in that** the sequence for the foreign protein comprises a sequence for the human serum albumin cDNA.

33. A transfected eukaryotic cell line according to claim 19, **characterised in that** it expresses human serum albumin.

## Revendications

1. Plasmide d'expression qui contient une unité de transcription/traduction dicistronique laquelle comprend une séquence pour une protéine étrangère, un site de liaison interne au ribosome et une séquence pour une protéine de fusion, la protéine de fusion contenant au moins un marqueur de sélection et au moins un marqueur d'amplification, le marqueur d'amplification étant la dihydrofolate réductase.

2. Plasmide d'expression selon la revendication 1, **caractérisé en ce que** le site de liaison interne au ribosome est la région 5' non traduite du virus de l'encéphalomyocardite (EMCV 5'UTR).

3. Plasmide d'expression selon la revendication 1 ou 2, **caractérisé en ce que** la séquence codante pour la protéine étrangère se trouve en 5' du site de liaison interne au ribosome et **en ce que** la séquence codante pour la protéine de fusion se trouve en 3' du site de liaison interne au ribosome.

4. Plasmide d'expression selon l'une des revendications 1 à 3, **caractérisé en ce qu'**il comprend un promoteur qui est le promoteur CMV, le promoteur SV 40 ou le promoteur β-actine humain.

5. Plasmide d'expression selon l'une des revendications 1 à 4, **caractérisé en ce que** l'unité de transcription/traduction dicistronique contient en outre un intron, de préférence l'intron de l'antigène t de SV40, l'intron 16s/19s ou le premier intron du gène de la β-actine humain, et un signal de polyadénylation, de préférence celui de l'unité de transcription précoce ou tardive du virus SV40.

6. Plasmide d'expression selon l'une des revendications 1 à 5, **caractérisé en ce que** la séquence de la protéine de fusion est constituée de deux séquences partielles, à savoir le gène de la dihydrofolate réductase, et d'un gène marqueur de sélection, de préférence le gène de l'hygromycine B-phosphotransférase.

7. Plasmide d'expression selon l'une des revendications 1 à 6, **caractérisé en ce que** le marqueur de sélection / amplification - protéine de fusion est bifonctionnel et **en ce que** la séquence codant la protéine de fusion est construite de telle sorte que la séquence partielle codante en 5' ne comprenne pas le codon stop et que la séquence partielle codante en 3' ne comprenne le cas échéant pas le codon start.

8. Plasmide d'expression selon l'une des revendications 1 à 6, **caractérisé en ce que** les séquences codantes des deux parties de protéine de la protéine de fusion sont séparées par un espaceur, en particulier par un espaceur long de 15 nucléotides.

9. Plasmide d'expression selon la revendication 8, **caractérisé en ce que** la séquence d'espaceur code 5 résidus glycine et présente la séquence GGA GGC GGG GGT GGA (SEQ ID n°2).

10. Plasmide d'expression selon la revendication 8, **caractérisé en ce que** la séquence d'espaceur code 5 résidus proline et présente la séquence CCA CCC CCG CCT CCA (SEQ ID n°1).

11. Plasmide d'expression selon l'une des revendications 1 à 10, **caractérisé en ce que** la séquence pour la protéine étrangère comprend une séquence pour une protéine plasmatique humaine ou une protéine virale ou un dérivé ou fragment d'une telle protéine.

12. Plasmide d'expression selon la revendication 11, **caractérisé en ce que** la séquence pour la protéine étrangère comprend une séquence pour l'ADNc de la prothrombine humaine.

13. Plasmide d'expression selon la revendication 11, **caractérisé en ce que** la séquence pour la protéine étrangère comprend une séquence pour l'ADNc du facteur VIII humain.

14. Plasmide d'expression selon la revendication 11, **caractérisé en ce que** la séquence pour la protéine étrangère comprend une séquence pour le mutant de délétion dB928 du facteur VIII humain.

15. Plasmide d'expression selon la revendication 11, **caractérisé en ce que** la séquence pour la protéine étrangère comprend une séquence pour l'ADNc du facteur XI humain.

16. Plasmide d'expression selon la revendication 11, **caractérisé en ce que** la séquence pour la protéine étrangère comprend une séquence pour l'ADNc de la protéine C humaine.

17. Plasmide d'expression selon la revendication 11, **caractérisé en ce que** la séquence pour la protéine étrangère comprend une séquence pour l'ADNc du facteur de Willebrand humain.

18. Plasmide d'expression selon l'une quelconque des revendications 1 à 17, **caractérisé en ce qu'**il comprend une ou plusieurs cassettes d'expression lesquelles contiennent les séquences d'ADN SEQ ID n°6, SEQ ID n°7 ou SEQ ID n°8.

19. Lignée cellulaire eucaryote transfectée, de préférence choisie parmi les lignées cellulaires CHO, 293 ou des lignées de cellules hépatiques humaines, comme SK-HEP-1 ou Chang Liver, qui est transfectée avec un plasmide d'expression selon l'une des revendications 1 à 18 et qui exprime une protéine étrangère.

20. Lignée cellulaire eucaryote transfectée selon la revendication 19, **caractérisée en ce qu'**elle exprime la prothrombine humaine.

21. Lignée cellulaire eucaryote transfectée selon la revendication 19, **caractérisée en ce qu'**elle exprime le facteur VIII humain.

22. Lignée cellulaire eucaryote transfectée selon la revendication 19, **caractérisée en ce qu'**elle exprime le mutant de délétion dB928 du facteur VIII humain.

23. Lignée cellulaire eucaryote transfectée selon la revendication 19, **caractérisée en ce qu'**elle exprime le facteur IX humain.

24. Lignée cellulaire eucaryote transfectée selon la revendication 19, **caractérisée en ce qu'**elle exprime la protéine C humaine.

25. Lignée cellulaire eucaryote transfectée selon la revendication 19, **caractérisée en ce qu'**elle exprime le facteur de Willebrand humain.

26. Procédé de fabrication de protéines étrangères, **caractérisé en ce qu'**une lignée cellulaire eucaryote est transfectée avec un plasmide d'expression selon l'une des revendications 1 à 18, **en ce que** les clones obtenus sont isolés par un processus de sélection sous le contrôle d'un marqueur de sélection et sont alors de préférence simultanément amplifiés, **en ce qu'**a lieu ensuite une amplification supplémentaire sous le contrôle du marqueur d'amplification, la protéine étrangère étant alors exprimée et récoltée.

27. Procédé selon la revendication 26, **caractérisé en ce que** le processus de sélection a lieu en utilisant l'hygromycine B et **en ce que** l'amplification supplémentaire a lieu en utilisant le méthotrexate.

28. Procédé selon la revendication 26 ou 27, **caractérisé en ce que**, comme lignées cellulaires CHO, 293 ou des lignées de cellules hépatiques humaines, comme SK-HEP-1 ou Chang Liver, sont transfectées avec un plasmide d'expression selon l'une des revendications 1 à 18.

29. Procédé selon l'une des revendications 26 à 28, **caractérisé en ce que** des facteurs de coagulation sanguine ou des protéines virales recombinants sont produits.

30. Procédé selon l'une des revendications 26 à 29, **caractérisé en ce que** la prothrombine humaine recombinante, le facteur VIII humain recombinant, FVIIIdB928 humain recombinant, le facteur IX humain recombinant, la protéine C humaine recombinante, l'albumine sérique humaine recombinante ou le facteur de Willebrand humain recombinant sont produits.

31. Utilisation de cellules SK-Hep-1, qui sont transfectées avec un plasmide d'expression selon l'une des revendications 1 à 18 et qui expriment une protéine étrangère, comme véhicule d'expression pour la prothrombine, le facteur VIII, le facteur VIIIdB928, le facteur IX, la protéine C, le facteur de Willebrand et/ou l'albumine sérique.

32. Plasmide d'expression selon la revendication 11, **caractérisé en ce que** la séquence pour la protéine étrangère comprend une séquence pour l'ADNc de l'albumine sérique humaine.

33. Lignée cellulaire eucaryote transfectée selon la revendication 19, **caractérisée en ce qu'**elle exprime l'albumine sérique humaine.
